(19) Europäisches Patentamt
European Patent Office
Office européen des brevets

(11) EP 4 186 509 A1

(12) EUROPEAN PATENT APPLICATION

(43) Date of publication:
31.05.2023 Bulletin 2023/22

(21) Application number: 21210661.1

(22) Date of filing: 26.11.2021

(51) International Patent Classification (IPC):
*A61K 31/55* (2006.01) *A61P 25/28* (2006.01)
*A61P 43/00* (2006.01)

(52) Cooperative Patent Classification (CPC):
**A61K 31/55; A61P 25/28; A61P 43/00;**
A61K 9/0043

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Alpha Cognition Inc.**
**Vancouver, British Columbia V6B 6L2 (CA)**

(72) Inventor: **KAY, Denis G.**
**Charlottetown, C1A 4P3 (CA)**

(74) Representative: **Hertin und Partner**
**Rechts- und Patentanwälte PartG mbB**
**Kurfürstendamm 54/55**
**10707 Berlin (DE)**

(54) **ALPHA-1062 FOR TREATING TRAUMATIC BRAIN INJURY**

(57) The invention relates to a pharmaceutical composition comprising a compound Alpha-1062 or salt thereof, or to a compound Alpha-1062 or salt thereof, for use in the treatment of confirmed or suspected traumatic brain injury (TBI) in a subject. In embodiments, the composition is administered transmucosally, such as intranasally, and/or the composition is self-preserving and anti-microbial. The invention also relates to a multi-use dispenser configured for intranasal or transmucosal administration of a pharmaceutical composition comprising Alpha-1062 or salt thereof. The invention further relates to a method of treating traumatic brain injury (TBI) in a subject comprising administering a therapeutically effective amount of a pharmaceutical composition comprising Alpha-1062 or salt thereof.

Fig. 9

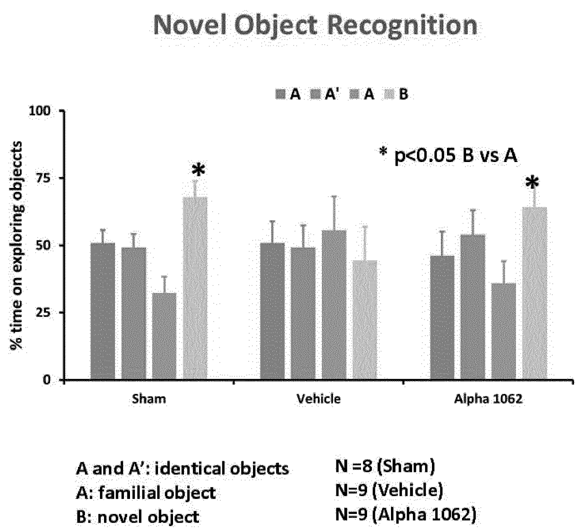


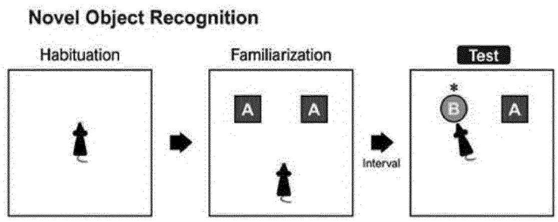




Processed by Luminess, 75001 PARIS (FR)

## Description

The present invention is in the field of compositions, formulations and dispensing devices for pharmaceutical agents, in addition to medical methods.

The invention relates to a pharmaceutical composition comprising a compound Alpha-1062 or salt thereof, or to a compound Alpha-1062 or salt thereof, for use in the treatment of confirmed or suspected traumatic brain injury (TBI) in a subject. In embodiments, the composition is administered transmucosally, such as intranasally, and/or the composition is self-preserving and anti-microbial. The invention also relates to a multi-use dispenser configured for intranasal or transmucosal administration of a pharmaceutical composition comprising Alpha-1062 or salt thereof. The invention further relates to a method of treating traumatic brain injury (TBI) in a subject comprising administering a therapeutically effective amount of a pharmaceutical composition comprising Alpha-1062 or salt thereof.

## BACKGROUND OF THE INVENTION

Traumatic brain injury (TBI) is a global public health problem and a leading cause of death and disability. TBI is clinically classified as mild, moderate, or severe at the time of injury, typically using a Glasgow Coma Scale (GCS). Severe TBI can result in mortality rates as high as 30-40%. Survivors experience a substantial burden of physical, psychiatric, emotional and cognitive disabilities which disrupt the lives of individuals and their families. Such disabilities are not restricted to severe cases, but also occur frequently after moderate or mild TBI.

The effects and outcome of TBI are not only influenced by the severity of injury but also by factors such as repetitive incidents and age. TBI is attributed to a variety of injury mechanisms, including falls, automobile or other types of traffic injuries, sports injuries, and interpersonal physical or other violence (e.g., explosion injury). In terms of age, TBI shows a bimodal distribution with the highest incidence in the youngest and oldest age groups. These age groups are likely to be more prone to TBI due to the increased risk of various causes of injury, such as falls in children under 4 years of age and in older people over 75 years of age, and motor vehicle accidents in adults.

Sport-related TBI is increasingly being described as a chronic health condition, as evidence suggests that the health effects of TBI can linger over time, especially in those who suffer from moderate to severe TBI. The majority of patients suffering a mild TBI (mTBI) recover quickly, but effects of mTBI can last for a year or more. Symptoms that are often associated with chronic effects include cognitive deficits, such as attention deficits, memory problems, and executive dysfunction or even social deficits. Long term effects of mTBI can range from impaired social inference - the interpretation of verbal and nonverbal social cues - to psychological symptoms such as anxiety, or physical symptoms such as fatigue, balance and coordination problems. mTBI can be associated with persistent headaches, vestibular dysfunction, depression, and cognitive complaints. The cumulative effects can lead to symptoms such as cognitive, behavioral, mood, and motor disorders.

Repeated injuries have been studied most thoroughly in contact sports such as American football, where the athletes have an increased risk of death from suicide, decreased cognitive functions, macrostructural, microstructural, functional and neurochemical changes and an increased risk of death from neurodegenerative causes such as dementia or Alzheimer’s disease. Another cause for TBI can be blast-related TBI in military personnel (Haarbauer-Krupa et al., J Neurotrauma, 2021; Brazinova et al., J Neurotrauma, 2021; Center-TBI project; American Association of Neurological Surgions (AANS)).

As of today, no curative treatment for TBI itself is available. Current medications aim to reduce secondary brain damage occurring as downstream effects or symptoms of TBI. Such drugs comprise anti-seizure drugs for patients experiencing seizures after moderate or severe TBI, coma-inducing drugs, which aim to reduce the oxygen consumption of the damaged brain and diuretics that can reduce the pressure inside the brain. Other available treatment options for TBI are only surgical interventions, physical and mental rest or rehabilitation treatment targeting chronical effects of TBI.

Galantamine and derivatives thereof have been suggested for the treatment of neurodegenerative diseases, such as Alzheimer’s and dementia. Unfortunately, similar to other cholinesterase inhibitors, galantamine has a clinically significant level of mechanism-based gastro-intestinal (GI) side effects, including nausea, vomiting and diarrhea (Loy C et al., Galantamine for Alzheimer’s disease and mild cognitive impairment. Cochrane Database of Systematic Reviews 2006). To accommodate patients to these side effects, cholinesterase inhibitors usually are initially administered at a low (non-efficacious) dose, with the dose being carefully up-titrated to an efficacious one, within a period of months. Moreover, the maintenance dose often is adjusted to what the patients experience as an acceptable level of GI side effects, making it likely that most, if not all, patients never achieve treatment with the most effective dose. Cholinesterase inhibitors such as galantamine are therefore considered suboptimal in addressing TBI, where immediate efficacious doses would be preferred.

To enhance the lipophilicity of acetylcholinesterase inhibitors and improve their passage through mucosal tissue, hydrophobic side chains have been appended to the basic alkaloid structures. Galantamine derivatives and prodrugs are described in EP1940817, WO2009/127218 and US2009/0253654.

The galantamine pro-drug Alpha-1062, is a benzoic acid or benzoate ester of galantamine ((4aS,6R,8aS)-5,6,9,10,11,12-hexahydro-3-methoxy-11-methyl-4aH-[1]benzofuro[3a,3,2-ef][2]benzazepin-6-ol benzoate). It was developed to enhance the hydrophobicity of galantamine. Alpha-1062 exhibits essentially no pharmacological activity until it is cleaved by esterases, resulting in the release of galantamine.

WO 2014/016430 discloses transmucosal administration of Alpha-1062 via intranasal, buccal or sublingual modes, in addition to various formulations and salts of Alpha-1062, including for example lactate, gluconate, maleate and saccharate salts.

The first nasal spray pumps for intranasal administration of liquid compositions were developed approximately 50 years ago and replaced the previous step by step droppers and pipettes. Nasal spray pumps are now widely used to moisturize the nasal mucosa using saline solutions, as nasal preparations for the administration of topically acting drugs, e.g. nasal decongestants, or for the non-invasive administration of substances which need to reach systemic circulation, e.g. antimigraine medication or hormones (Marx and Birkhoff, "Multi-Dose Container for Nasal and Ophthalmic Drugs: A Preservative Free Future?" in Drug Development - A Case Study Based Insight into Modern Strategies, ed. Chris Rundfeldt, 2011).

For many disease indications, multi-dose devices are cost effective and convenient means to provide the safety and precision in administration of active agents that regulatory bodies require. However, until now, most medications administered transmucosally, commonly as solutions, emulsions or suspensions, contain a preservative to support long storage times and proper in-use stability for multi-use or multi-dose dispensers. However, despite the advantages of nasal administration, the use of preservatives in nasal sprays is controversial. Reports suggest that preservatives in nasal sprays might increase the risk of adverse events for patients.

As no medicaments are available for the first-line treatment of TBI that could reduce or prevent secondary downstream injuries, and short and/or long-term symptoms of TBI, such as cognitive and neurological impairments, the provision of effective treatments for TBI is urgently needed.

## SUMMARY OF THE INVENTION

In light of the prior art the technical problem underlying the invention was the provision of improved or alternative means for treating traumatic brain injury (TBI). Another problem underlying the invention was the provision of a convenient, multi-use and preferably preservative-free formulation suitable for administering an efficacious amount of an active pharmaceutical agent in treating TBI.

This problem is solved by the features of the independent claims. Preferred embodiments of the present invention are provided by the dependent claims.

The invention therefore relates to a pharmaceutical composition comprising a compound Alpha-1062 or salt thereof for use in the treatment of confirmed or suspected traumatic brain injury (TBI) in a subject.

In other embodiments or aspects, the invention relates to a compound Alpha-1062 or salt thereof, for use in the treatment of confirmed or suspected traumatic brain injury (TBI) in a subject

In other embodiments or aspects, the invention further relates to a method of treating traumatic brain injury (TBI) in a subject comprising administering a pharmaceutically effective amount of a pharmaceutical composition comprising Alpha-1062 or salt thereof

The present invention is based on the surprising finding that Alpha-1062 shows efficacy in treating TBI. As discussed in more detail below, an exemplary 35-day study using a controlled cortical impact injury model was conducted, indicating the effectiveness of Alpha-1062. The positive therapeutic effects on TBI may include, without limitation, improved functional and histological outcomes after traumatic brain injury (TBI). To the knowledge of the inventors, the improvements observed could not have been predicted from the prior art with respect to the TBI group.

In one embodiment the composition is in liquid form.

In embodiments, the liquid is a solution, emulsion or a suspension.

In another embodiment the composition is in solid form.

In embodiments, the solid form is a tablet, pill, film, lozenge or capsule.

The invention also relates to a pharmaceutical composition comprising a compound Alpha-1062 or salt thereof for use in the treatment of confirmed or suspected traumatic brain injury (TBI) in a subject, wherein the composition is administered transmucosally.

In embodiments, the composition is administered intranasally.

In embodiments, the composition is administered to the oral cavity.

In embodiments, the composition is administered sub-lingually or buccally.

Advantageous transport properties can be achieved for pro-drugs when administered by intravenous injection, but less well, or only to a small extent, when they are administered orally, as e.g. tablets, for systemic administration. This is often because pro-drug esters are instable in acidic environments (such as exists in the stomach) and may be cleaved enzymatically in many tissues, including in the intestines and in the liver (first-pass effect).

In light of these findings and the problems of the administration methods of the prior art, and in order to take advantage of Alpha-1062 in the treatment of CNS diseases, in some embodiments the invention makes use of administration routes that avoid the gastro-intestinal tract and the first-pass effect (loss of a certain percentage of a drug due to the metabolism in the gut and liver). These routes of administration for Alpha-1062 provide brain delivery of the active drug galantamine at similar levels as intravenous injection of Alpha-1062. The invention therefore employs in some embodiments pharmaceutical formulations to be used for the selected routes of administration that optimize rapid resorption and uptake of Alpha-1062.

Of note, is that transmucosal administration of galantamine, as in the prior art, provides no such enhancement, as galantamine is not susceptible to cleavage by endogenous esterases. A surprising concept of transmucosal administration of Alpha-1062in treating TBI is based on the avoidance of cleavage of the prodrug post-administration but before partition via the BBB, thereby enhancing brain transport and increased relative concentration of the active substance after cleavage, which under conditions of the proposed routes of administration and drug formulations, occurs significantly in the brain.

Hence, the invention provides improved means and formulations that can be administrated immediately after the occurrence of brain trauma, in an effective and easy-to-use form that shows low side effects and enables good patient compliance.

As shown in the Examples, the inventors developed application and treatment methods that surprisingly reduce the short- and long-term effects of TBI, such as impaired cognitive, neurological and motoric functions. The formulations and compounds according to the invention show surprisingly beneficial effects in the treatment of subjects, which have suffered TBI or potentially other brain traumata. The treatment of subjects that have suffered a TBI event with formulations and compounds according to the present invention comprising Alpha-1062 or salts thereof results in a better recovery of motor skills, memory and other cognitive functions, in comparison to vehicle treatment. The treatment of subjects suffering from TBI with Alpha-1062 or salts thereof according to the present invention appears to cause superior restoration of motor and memory skills and other cognitive functions, which are comparable to skills in healthy individuals that did not suffer from TBI. This surprising effect is complemented by transmucosal administration of the compounds and compositions according to the invention, which facilitates not only improved patient compliance due to its easy administration and its reduced adverse effects, but also achieves a good efficacy of the compound due to the direct delivery of the compound to the brain (avoidance of first pass effect). This surprising effect may represent a significant improvement in the current treatment options for TBI and is evidenced by the experimental results shown in the Examples.

Accordingly, the present invention provides new and surprising solutions for treating, reducing and/or preventing (or reducing the risk of) TBI-related short and long-term symptoms, such as acute and/or chronic neurological and cognitive impairment.

The invention therefore relates in one embodiment to Alpha-1062 for use as a medicament in the treatment of TBI as described herein, wherein transmucosal administration is configured to avoid and/or reduce immediate post-administration cleavage of the ester group of said substance by endogenous esterases, e.g. during and/or after absorption from the gastrointestinal tract.

This aspect of the present invention, namely addressing the relatively low stability of the ester moiety of Alpha-1062 in the gastro-intestinal tract and liver, represents a novel technical effect in the treatment for TBI not previously disclosed or suggested in the art.

The avoidance of in vivo esterase cleavage - with regard to the improvements obtained by transmucosal administration and the enhanced delivery of the compounds and salts described herein - enables treatment of patients who would previously have avoided or discontinued treatment with ChE inhibitors due to strong gastro-intestinal side effects associated with orally administered tablets. The improved brain delivery via transmucosal administration, in particular of high concentration aqueous solutions of Alpha-1062 salts administered nasally, permits dosage regimes which were previously challenging or impractical, with respect to galantamine (due to significant side effects) or Alpha-1062 (due to in vivo degradation via the oral route).

The invention also relates to a pharmaceutical composition comprising a compound Alpha-1062 or salt thereof for use in the treatment of confirmed or suspected traumatic brain injury (TBI) in a subject, wherein the composition is self-preserving and anti-microbial, wherein preferably the composition is essentially absent of additional antimicrobial preservatives.

As described in detail below, the invention is based on the surprising finding of beneficial effects of the use of Alpha-1062 in the treatment of TBI. No indication is evident in the prior art prior that this agent (Alpha-1062) has anti-microbial properties. As is described in the introduction, Alpha-1062 was known to exhibit an effect against cognitive impairment by acting as a pro-drug of galantamine. No suggestion is evident in the art that galantamine or its prodrug Alpha-1062 could exhibit the property of actively reducing CFU/mL of pathogenic bacteria, yeast and fungi, as demonstrated according to the USP 51 test.

The identification of this novel property (anti-microbial effect) of a known substance (Alpha-1062) opens new clinical perspectives and creates a new clinical situation when considering either formulating or administering the substance. New patient populations have been enabled due to the discovery of the novel properties, and new dosage regimes and formulation options may now be employed, based on the surprising property.

The provision of a self-preserving Alpha-1062 composition, preferably as a liquid, or more preferably as an emulsion or solution, such as in the form of multi-use dispensers without additional preservatives, enables reduced side effects, for example side effects caused by nasal administration of a solution that comprises an additional preservative. Storage properties of any given formulation may now be improved, i.e. longer storage times may be evident due to the discovery of the anti-microbial properties.

In some embodiments, reduced side effects may be achieved. Such reduced side effects (i.e. as caused by preservatives) may relate to nasal irritation, such as but without limitation to, increased mucosal swelling and nasal hyper-reactivity, type IV hypersensitivity, decrease of mucociliary clearance, and nasal mucosa dysplasia.

In some embodiments, the composition of the invention does not comprise one or more, preferably does not comprise any, additional preservative(s), selected from the list consisting of benzalkonium (preferably benzalkonium chloride), benzyl alcohol, thimerosal (merthiolate), edetate disodium, monobasic sodium phosphate, providone, dibasic sodium phosphate, disodium eta, potassium phosphate monobasic, iodine, phenylcarbinol and sodium silicoaluminate.

Furthermore, the provision of a self-preserving Alpha-1062 solution, such as in the form of multi-use dispensers without additional preservatives, enables good patient compliance compared to single-use or single-dose administration, as described in the art. The provision of a simple spray dispenser, capable of multiple uses, but without preservatives, is a simple and low side effect-option for patients. Firstly, low mucosal or nasal irritation should lead to enhanced patient compliance, as discomfort, e.g., in the nose and/or mouth, is reduced. Secondly, using an easy-to-use dispenser and keeping this single dispenser over time, for multiple uses, makes administration simpler for cognitive and/or neurologically impaired and/or elderly subjects, or if the treatment is administered to infants or children.

In contrast, the daily administration of tablets intended for swallowing or single-dose dispensers is associated with additional complication, requiring a patient, who is potentially a child or cognitive and/or neurologically impaired, to either swallow tablets or to continually open, apply and purchase single dose dispensers, leading to complications and delivery burden. Consequently, the herein described administration forms of the compositions and compounds according to the present invention reduce adverse effects and improve the patient compliance in the treatment of TBI, resulting in further improved treatment outcomes of TBI, as regular and/or long-term administration of the compounds according to the invention is ensured or at least supported.

The multi-use dispenser of the invention may therefore be maintained in first-aid kits, or the first response equipment of emergency medical personnel. The anti-microbial features of Alpha-1062, in combination with its efficacious effect in treating TBI and/or symptoms thereof, enables a multi-use dispenser that does not require frequent replenishment or replacement, making it an ideal active agent in e.g. pre-prepared first aid medical kits.

In one embodiment, the pharmaceutical composition comprising a compound Alpha-1062 or salt thereof for use in the treatment of confirmed or suspected traumatic brain injury (TBI) in a subject comprises a sufficient amount of Alpha-1062 to have an anti-microbial effect. Such an antimicrobial effect may in some embodiments refer to an antimicrobial effect in the pharmaceutical formulation, such as in maintaining a relatively microbial free or low-microbe environment within the formulation, or to in vivo effects, post-administration, leading to a reduction of microbial load in the area of administration.

In one embodiment, the pharmaceutical composition comprising Alpha-1062 or salt thereof for use in the treatment of confirmed or suspected traumatic brain injury (TBI) in a subject is characterized in that the compound Alpha-1062 or salt thereof is present at a concentration of 1 to 200 mg/mL, preferably between 5 and 100 mg/mL.

In some embodiments, the Alpha-1062 or salt thereof are present in the composition at a concentration of 1 to 500 mg/mL, preferably between 1 and 400 mg/mL, more preferably between 1 and 300 mg/mL, preferably between 1 and 200 mg/mL, preferably between 5 and 100 mg/mL.

In some embodiments, the Alpha-1062 or salt thereof are present at about 5 mg/mL, or 10, 15, 20, 25, 30, 35, 40, 45, 50, 55, 60, 65, 70, 75, 80, 85, 90, 95, 100, 110, 120, 130, 140, or at about 150 mg/mL. Ranges constructed form any given of the afore-mentioned values are also contemplated.

In some embodiments, the Alpha-1062 is present in the composition as a salt, preferably a lactate, gluconate, maleate or saccharate salt. Generation of Alpha-1062 salts are described in the art, e.g. in WO 2014/016430, and can be carried out without undue burden.

In some embodiments, the salt comprises stoichiometric and/or non-stoichiometric salts and/or hydrates of the chemical substances according to Alpha-1062, whereby the salt is preferably described as:

$$\text{Alpha-1062} \cdot n\,HX \cdot m\,H_2O,$$

wherein n and m = 0 - 5, and n and m can be the same or different, and HX is an acid, selected preferably from lactic acid, gluconic acid, maleic acid or saccharic acid.

In some embodiments, other acids may be employed for the Alpha-1062 salt formation.

Acids useful for preparing the pharmaceutically acceptable acid addition salts according to the invention include inorganic acids and organic acids, such as sulfamic, amidosulfonic, 1,2-ethanedisulfonic, 2-ethylsuccinic, 2-hydroxyethanesulfonic, 3-hydroxynaphthoic, acetic, benzoic, benzenesulfonic acid, carboxylic, ethylenediamine tetraacetic acid, camphorsulfonic, citric, dodecylsulfonic, ethanesulfonic, ethenesulfonic, ethylenediamine tetraacetic, fumaric, glubionic, glucoheptonic, gluconic, glutamic, hexylresorcinic, hydrobromic, hydrochloric, isethionoc, (bi)carbonic, tartaric, hydriodic, lactic, lactobionic, laevulinic, laurylsulfuric, lipoic, malic, maleic, malonic, mandelic, methanesulfonic, mucic, naphthalenesulfonic, nitric, oxalic, pamoic, pantothenic, perchloric, phosphoric, polygalacturonic, pectic, propionic, salicylic, succinic or sulfuric acid, p-tuluenesulfonic, wherein hydrochloric, hydrobromic, sulfuric, nitric, phosphoric and perchloric acids, as well as tartaric, citric, acetic, succinic, maleic, fumaric and oxalic acids.

It was surprising, that embodiments of the pharmaceutically applicable solutions of Alpha-1062 salts fulfilled the criteria for appropriate stability, concentration, pH, osmolarity and nasal mucosal tolerance in solution, for intranasal application in the treatment of TBI.

In one embodiment, the Alpha-1062 salt has solubility in water of at least 10%, preferably > 20%, or more preferably >30% weight per volume (w/v). The high solubility enables higher concentrations of the compound to be administered in smaller volumes, thereby further enhancing administration via e.g. transmucosal administration.

The preferred embodiment of transmucosal administration represents a beneficial mode of delivery due to a combination of factors. The enhanced solubility of Alpha-1062 salts allows higher concentrations of Alpha-1062 to be administered, thereby enabling larger amounts of the active substance after cleavage (galantamine) to be active in the brain. In embodiments the prodrug properties of Alpha-1062 are exploited and enhanced by the transmucosal application of the Alpha-1062 salts.

The invention therefore also relates to a method for treating a confirmed or suspected brain injury associated with cognitive and/or neurological impairment in a subject, the method comprising administering a therapeutically effective amount of Alpha-1062 or salt thereof to a subject in need thereof.

In one embodiment, the pharmaceutical composition of the invention has the compound Alpha-1062 present as a gluconate salt, preferably at a concentration of 50-100 mg/mL, more preferably 70-90 mg/mL, or alternatively at a concentration disclosed herein. A skilled person is aware of varying the concentration of the active agent according to common practice.

In one embodiment, the pharmaceutical composition of the invention is characterized in that the compound Alpha-1062 is present at a concentration sufficient to reduce $1\times10^4$ to $1\times10^6$ colony-forming units per mL (CFU/mL) of *Escherichia coli, Pseudomonas aeruginosa, Staphylococcus aureus, Candida albicans* and/or *Aspergillus brasiliensis* to <100 CFU/mL, preferably <10 CFU/mL, within 14 days treatment, according to the United States Pharmacopeia Chapter 51 preservative test (USP 51). Alpha-1062 shows anti-microbial activity against both gram-positive and gram-negative bacteria, pathogenic yeasts and fungi, thereby indicating a broad-spectrum anti-microbial effect. As there is no suggestion in the prior art that Alpha-1062 could exhibit such a broad and effective anti-microbial activity and that treatment of TBI could benefit from it, the exploitation of this property of Alpha-1062 in the treatment of TBI represents therefore a surprising and unexpected finding with practical utility.

In some embodiments, the liquid composition is stable, i.e. exhibits sufficient drug or pro-drug stability and low microbial loads, over extended periods. In some embodiments, the extended period is for about 1 week, or 1, 2, 3, 4, 5, 6, 7, 8 weeks, or longer. In some embodiments, the composition is stable for about 1 month, or 2, 3, 4, 5, 6, 7, 8, 9, 10, 11, or 12 months. During this time, the composition may be administered via multi-use device of the invention, or in storage. In some cases, storage of the composition is possible (preferably without use by a subject) for about 1, 2, 3, 4 or 5 years.

In embodiments the composition is present in a multi-use dispenser configured for transmucosal administration.

In embodiments the composition is present in a single-use dispenser configured for transmucosal administration.

In embodiments the composition is present in a suitable metered dose device such as an atomizer, sprayer, pump spray, dropper, squeeze tube, squeeze bottle, pipette, ampule, nasal cannula, metered dose device, nasal spray inhaler, nasal continuous positive air pressure device, and/or breath actuated bi-directional delivery device.

In one embodiment, a therapeutically effective amount of the compound is administered using a suitable metered multi-use dose device or dispenser, such as a multi-use atomizer, multi-use sprayer, multi-use pump spray, multi-use dropper, multi-use squeeze tube or bottle multi-use metered dose device or multi-use nasal sprayer or inhaler.

In one embodiment, a therapeutically effective amount of the compound is administered using a suitable single-use dose device or dispenser. In embodiments the single-use dose device or dispenser is selected from the group comprising a single use dropper, a single-use squeeze tube or bottle, and a single dose powder dispenser.

In one embodiment, a therapeutically effective amount of the Alpha-1062 or salt thereof is administered to the oral cavity.

In one embodiment, a therapeutically effective amount of the compound Alpha-1062 or salt thereof is administered under the tongue (sub-lingual) by dispensing an amount of compound, preferably in the form of a solution or

emulsion, from a multi-use dispenser, and/or by spraying the underside of the tongue with a preselected volume of a liquid composition, preferably a solution or emulsion, from a multi-use dispenser comprising the compound or salt thereof.

In one embodiment, a therapeutically effective amount of the compound or salt thereof is administered to the buccal vestibule inside the mouth between the cheek and the gums, preferably as a solution or emulsion, from a multi-use dispenser.

In a further aspect of the invention, in the non-limiting context of a multi-use dispenser, the invention combines the properties, features and advantages of multi-use transmucosal delivery devices with the anti-microbial property of Alpha-1062 or salts thereof. Accordingly, the multi-use dispenser according to the invention configured for transmucosal administration of a pharmaceutical composition in the form of a liquid, comprises a self-preserving anti-microbial solution as described herein with Alpha-1062 or salt thereof.

In one embodiment the administration to the oral cavity is carried out by placing one or more drops of a solution or emulsion to the oral cavity, by spraying a preselected volume of a liquid composition to the oral cavity, or by administering a sub-lingual tablet, film formulation, lozenge, orally disintegrating or orally dispersing tablet to the oral cavity.

In one embodiment, a therapeutically effective amount of the compound is administered to the buccal vestibule inside the mouth between the cheek and the gums, preferably as a solution or emulsion, from a multi-use dispenser.

In one embodiment, the multi-use dispenser is configured for intranasal administration.

In one embodiment, the multi-use dispenser is configured for administration to the oral cavity.

In one embodiment, the multi-use dispenser is configured for transmucosal administration.

In one embodiment, the multi-use dispenser as described herein is for use in the treatment of confirmed or suspected traumatic brain injury (TBI) in a subject (or for use in a corresponding method of treatment), wherein the treatment comprises administering multiple doses of the pharmaceutical composition, which is preferably self-preserving anti-microbial, as described herein, to the subject from the same dispenser.

Hence, in another aspect, the invention relates to a multi-use dispenser configured for intranasal administration of a pharmaceutical composition comprising Alpha-1062 or salt thereof in the form of a liquid, for use in the treatment of confirmed or suspected traumatic brain injury (TBI) in a subject.

Hence, in another aspect, the invention also relates to a multi-use dispenser configured for administration to the oral cavity of a pharmaceutical composition comprising Alpha-1062 or salt thereof in the form of a liquid, for use in the treatment of confirmed or suspected traumatic brain injury (TBI) in a subject.

In a further aspect, the invention relates to a single-use dispenser configured for intranasal administration of a pharmaceutical composition comprising Alpha-1062 or salt thereof in the form of a liquid, for use in the treatment of confirmed or suspected traumatic brain injury (TBI) in a subject.

In another aspect, the invention also relates to a single-use dispenser configured for administration to the oral cavity of a pharmaceutical composition comprising Alpha-1062 or salt thereof in the form of a liquid, for use in the treatment of confirmed or suspected traumatic brain injury (TBI) in a subject.

The following embodiments relate to both, the administration of a composition comprising a compound Alpha-1062 or salt thereof as disclosed herein, as well as to the herein disclosed pharmaceutical composition, to the single- and the multi-use dispenser.

In one embodiment of the multi-use dispenser configured for intranasal administration of a pharmaceutical composition comprising Alpha-1062 or salt thereof in the form of a liquid, for use in the treatment of confirmed or suspected traumatic brain injury (TBI) in a subject, the multi-use dispenser is a suitable metered multi-use dose device or dispenser, such as an inhaler, atomizer, sprayer, pump spray, dropper, squeeze tube, squeeze bottle, pipette, ampule, nasal cannula, metered dose device, nasal spray inhaler, nasal continuous positive air pressure device, breath actuated bi-directional delivery device a multi-use atomizer, multi-use sprayer, multi-use pump spray, multi-use dropper, multi-use squeeze tube or bottle, multi-use metered dose device and/or multi-use nasal sprayer.

In one embodiment of the multi-use dispenser configured for intranasal or transmucosal administration of a pharmaceutical composition comprising Alpha-1062 or salt thereof in the form of a liquid, for use in the treatment of confirmed or suspected traumatic brain injury (TBI) in a subject, the multi-use dispenser dispenses in one dosing event volumes of between 10 $\mu$l to 200 $\mu$l.

In one embodiment of the multi-use dispenser the dispenser further comprises different actuators, and/or neck finishes known in the art, such as screw-on, snap-on and crimp-on.

Preferred dispensers of the invention relate to any of the above-mentioned multi-use devices. In some embodiments, the dispensers available for example from Nemera (La Verpilliere, France) or Aptar Pharma (Illinois, USA) are preferred.

Nasal sprays can be contaminated through their drug delivery orifices, with bacterial contamination coming from the external environment or the patient, or from the air. Therefore, in some embodiments, to prevent contamination via air entering the device, nasal sprays may be employed in which a filter system is present that stops the entry of bacteria into the container. Airborne bacteria are typically around 0.3 $\mu$m, therefore appropriately sized filters may be selected. Furthermore, recent studies have demonstrated that bacterial transfer through filter membranes takes place

during filtration operations, even if the pore size is significantly smaller than the bacteria size. Therefore, in some embodiments, devices are employed that utilize a silicone membrane to filter the returning air.

In some embodiments, the multi-use dispenser employs a membrane (preferably of silicone) that prevents bacteria from entering the reservoir or pump device. In one embodiment of the multi-use dispenser according to the invention the multi-use dispenser comprises a spring-loaded tip seal mechanism, a filter membrane in the ventilation channel, a venting system with a silicone membrane, a permeable membrane and/or a silicone membrane to reduce the contamination of the composition comprised within the dispenser. In some embodiments, the multi-use dispenser employs a spring-loaded tip seal mechanism, thereby preventing microbes from entering the device between spray events. In some embodiments, the multi-use dispenser employs a metal-free fluid path, thereby preventing the oxidization of the formulation.

Instead of using preservatives, advanced spray dispenser technology represents an alternative way of keeping a nasal spray sterile, by preventing bacteria entering and contaminating the drug formulation. Such dispensers may also be employed in the present invention, thereby further reducing microbial burden in the multi-use dispenser. In combination, the anti-microbial properties of Alpha-1062 or salts thereof with the "preservative-free" dispenser technology, as described herein, and as is available to a skilled person from e.g. Nemera or Aptar, lead to unexpectedly good shelf life (either in storage or during usage) of a liquid composition comprising Alpha-1062 or salts thereof.

In one embodiment, the dispenser is configured for multiple individual spray events of 5 to 1000 μL, preferably 5 to 500 μL, more preferably 10 to 300 μL, more preferably 20 to 200 μL.

In one embodiment, the dispenser is configured for multiple individual spray events of about 5 μL, or 10, 15, 20, 25, 30, 35, 40, 45, 50, 55, 60, 65, 70, 75, 80, 85, 90, 95, 100, 120, 140, 160, 180, 200, 250, 300, 350, 400, 450 or 500 μL volume. Ranges constructed from any given of the afore-mentioned values are also contemplated.

In one embodiment, the dispenser comprises a total volume of composition of 1 to 500 mL, preferably 1 to 100 mL, more preferably 2 to 50 mL, such as for example about 5, 10 or 15 mL.

In one embodiment, the dispenser comprises about 1 mL, 2, 3, 4, 5, 6, 7, 8, 9, 10, 11, 12, 13, 14, 15, 16, 18, 20, 30, 40, 50, 60, 70, 80, 90, 100, 110, 120, 130, 140, 150, 160, 170, 180, 190, 200, 220, 240, 260, 280, 300, 350, 400, 450, or 500 mL volume. Ranges constructed from any given of the afore-mentioned values are also contemplated.

In some embodiments, the dispenser must comprise a sufficient amount of composition for administration of multiple doses. In some embodiments, the dispenser comprises a sufficient volume of composition for at least 2, or at least, or about 3, 4, 5, 6, 7, 8, 9, 10, 12, 14, 16, 18, 20, 25, 30, 35, 40, 45, 50, 60, 70, 80, 90 or about 100 individual doses, or more doses. Ranges constructed form any given of the afore-mentioned values are also contemplated.

In one embodiment, the dispenser is configured for multiple individual spray events of 20 to 200 μL, and wherein said dispenser comprises a total volume of solution of 2 to 50 mL.

In one embodiment, the multi-use dispenser is configured for and/or the Alpha-1062 or salt thereof is administered at a dose of 1 to 100 mg, one to three times daily, preferably for multiple days.

In one embodiment, the multi-use dispenser is configured for and/or the Alpha-1062 or salt thereof is administered at a dose of 2 to 40 mg, twice daily, preferably over multiple days.

In one embodiment, the multi-use dispenser is configured for and/or the Alpha-1062 or salt thereof is administered at a dosage of from 0.1 to 200 mg, 1 to 100 mg, preferably 2 to 40 mg, preferably from one to three times daily, more preferably twice daily, and even more preferably only once daily, over multiple days.

In one embodiment, the Alpha-1062 or salt thereof is administered intranasally as a 2 to 40% weight per volume (w/v) solution at an amount of 20 to 100 microliters in each of multiple nasal spray events, one to three times daily, over multiple days.

In one embodiment, the multi-use dispenser is configured for and/or the Alpha-1062 or salt thereof is administered, preferably intranasally, as an about 10% weight per volume (w/v) solution at an amount of about 50 microliters in each of multiple administration events, one to three times daily, preferably over multiple days.

In one embodiment, the multi-use dispenser is configured for and/or the Alpha-1062 or salt thereof is administered by intranasal, buccal or sublingual administration, preferably as a 2 to 40% weight per volume (w/v) solution, for example at an amount of 20 to 100 microliters, preferably multiple (intranasal or oral (sub-lingual/buccal)) administration events, for example from one to three times daily, preferably over multiple days.

In some embodiments, the liquid formulations are configured for any one or more of the above administration modes. A skilled person is aware of technical means employed in configuring compositions for specific modes of administration. For example, a composition configured for nasal administration may be formulated, packaged, or prepared in a different manner from compositions prepared for oral administration.

In embodiments of the invention the TBI is classified as mild TBI, moderate TBI, or severe TBI.

In the context of the use of Alpha-1062 or salts thereof in the treatment of confirmed or suspected TBI, the composition or compound can be administered to subjects immediately after an acute TBI event as well as to subjects who suffered a suspected or confirmed TBI event in the past. Hence, the use of Alpha-1062 or salts thereof refers in embodiments to the treatment and/or the prevention of acute and/or chronic symptoms and/or outcomes of TBI.

In embodiments, the invention therefore relates to a pharmaceutical composition comprising a compound Alpha-1062 or salt thereof for use in the treatment of confirmed or suspected traumatic brain injury (TBI) in a subject, wherein the subject that has or is suspected to suffer from TBI shows one or more symptoms selected from the group consisting of dizziness, balance problems, headaches, nausea, vomiting, light sensitivity, impaired memory, sleep abnormalities, impaired concentration and impaired vision.

In embodiments, the invention also relates to a pharmaceutical composition comprising a compound Alpha-1062 or salt thereof for use in the treatment of confirmed or suspected traumatic brain injury (TBI) in a subject, wherein the subject that has or is suspected to suffer from moderate or severe TBI shows one or more symptoms selected from the group consisting of weakness in arms and legs, balance and coordination problems, severe or increasing headaches, impaired sensory perception, impaired cognitive skills, impaired memory, impaired communication and learning, personality changes, behavior abnormalities and impaired vision and hearing.

In embodiments, the invention also relates to a pharmaceutical composition comprising a compound Alpha-1062 or salt thereof for use in the treatment of confirmed or suspected traumatic brain injury (TBI) in a subject, wherein the subject that has or is suspected to suffer from severe TBI shows one or more symptoms selected from the group consisting of paralysis, coma, loss of consciousness, dilated pupils, loss of cerebrospinal fluid from the ears or nose, loss of bowel and/or bladder control, breathing problems, slow pulse, breathing problems, slow breathing rate with an increase in blood pressure and droopy eyelid or facial weakness.

In embodiments, the invention also relates to a pharmaceutical composition comprising a compound Alpha-1062 or salt thereof for use in the treatment of confirmed or suspected traumatic brain injury (TBI) in a subject, wherein the traumatic brain injury (TBI) leads to or is accompanied by one or more injuries selected from the group consisting of a hematoma, contusion, intracerebral hemorrhage, subarachnoid hemorrhage, diffuse injuries, diffuse axonal injury, ischemia, primary brain injury and secondary brain injury.

The formulations and compounds according to the invention show the surprising beneficial effects in the treatment of TBI or potentially other brain traumata of reducing the severity and/or preventing (reducing the risk of) the occurrence of short and long-term symptoms of TBI. As shown in the Examples herein, the transmucosal administration of compositions comprising Alpha-1062 or salts thereof, according to the present invention, to subjects suffering from TBI or other brain traumata results in an improved recovery of motor skills, memory and other cognitive functions, when compared to vehicle treatment after TBI.

In embodiments, the traumatic brain injury (TBI) is caused by a closed head injury.

In embodiments, the traumatic brain injury (TBI) is caused by a penetrating head injury.

In embodiments, the traumatic brain injury (TBI) is caused by a head injury resulting from an incident selected from the group consisting of a fall, a motor vehicle-related incident, a strike or blow to the head from or against an object, a sports-related incident, interpersonal physical violence or violence by other means.

In embodiments, the subject that has or is suspected to suffer from TBI is an infant under the age of 4 years.

In embodiments, the subject is under 12 months of age.

In embodiments, the subject that has or is suspected to suffer from TBI is a child from 4 to 12 years of age,

In embodiments, the subject is an adolescent from 12 to 17 years of age.

In embodiments, the subject that has or is suspected to suffer from TBI is an adult, selected from an adult of between 18 and 65 years of age.

In embodiments, the subject is an (elderly) adult of over 65 years of age.

In embodiments, the invention relates to a pharmaceutical composition comprising a compound Alpha-1062 or salt thereof for use in the treatment of confirmed or suspected traumatic brain injury (TBI) in a subject, wherein the traumatic brain injury (TBI) is associated with trauma to the nasal cavity.

In embodiments, the invention relates to treatment of traumatic brain injury (TBI) and concurrent treatment and/or prevention of microbial infection of the nasal cavity.

In embodiments, the invention also relates to a pharmaceutical composition comprising a compound Alpha-1062 or salt thereof for use in the treatment of confirmed or suspected traumatic brain injury (TBI) in a subject, wherein the traumatic brain injury (TBI) is associated with disruption of the blood brain barrier.

In embodiments, the invention relates to treatment of traumatic brain injury (TBI) and concurrent treatment and/or prevention of microbial infection of the central nervous system.

The combination of therapeutic properties of Alpha-1062 including both neurological brain function improving properties and antimicrobial properties relates to an unexpected combination of benefits that make the invention ideally suited for TBI treatment, in particular in situations where first-responders or emergency medical personnel must treat quickly post-injury, but also to ongoing use during recovery. In particular, any brain trauma combined with injury to the nasal or oral cavity may be effectively treated using this combination of properties.

The invention therefore also relates to a method as described herein of treating confirmed or suspected traumatic brain injury (TBI) in a subject, wherein said subject suffers additionally from a microbial infection, wherein said method comprises administering Alpha-1062 or salt thereof to a subject in need thereof.

Alpha-1062 exhibits a strong anti-microbial effect against various pathogenic microbes. As such, the compound appears ideally suited for the treatment of TBI in a subject, wherein said subject suffers additionally from a microbial infection. In some embodiments, the microbial infection is associated with the TBI. In some embodiments, the infection is unrelated to the TBI. In some embodiments, the infection is a microbial infection, wherein the microbial infection is preferably a pathogenic microbial infection in a subject.

According to the preferred modes of administration, described herein, the compound will come into contact with various mucosal surfaces, which may be subject to an unwanted infection i.e. by a pathogen. The anti-microbial activity of the compound therefore provides a beneficial effect upon administration to a subject.

In embodiments, the invention also relates to a method for reducing incidence of or treating a traumatic brain injury (TBI) in an individual, comprising administering to the individual an effective amount of a compound of Alpha-1062 or a salt thereof.

In embodiments, the invention also relates to a method for reducing incidence of symptoms selected from the group consisting of dizziness, balance problems, headaches, nausea, vomiting, light sensitivity, impaired memory, sleep abnormalities, impaired concentration and impaired vision as a consequence of traumatic brain injury, or treating traumatic brain injury in a subject, comprising administering to the subject an effective amount of a compound of Alpha-1062 or a salt thereof.

In embodiments, the invention also relates to a method for reducing incidence of symptoms selected from the group consisting of weakness in arms and legs, balance and coordination problems, severe or increasing headaches, impaired sensory perception, impaired cognitive skills, impaired memory, impaired communication and learning, personality changes, behavior abnormalities and impaired vision and hearing as a consequence of traumatic brain injury, or treating traumatic brain injury in a subject, comprising administering to the subject an effective amount of a compound of Alpha-1062 or a salt thereof.

In embodiments, the invention relates to a method for reducing incidence of symptoms selected from the group consisting of paralysis, coma, loss of consciousness, dilated pupils, loss of cerebrospinal fluid from the ears or nose, loss of bowel and/or bladder control, breathing problems, slow pulse, breathing problems, slow breathing rate with an increase in blood pressure and droopy eyelid or facial weakness as a consequence of traumatic brain injury, or treating traumatic brain injury in a subject, comprising administering to the subject an effective amount of a compound of Alpha-1062 or a salt thereof.

In embodiments, the invention relates to a pharmaceutical composition comprising a compound Alpha-1062 or salt thereof and preferably one or more pharmaceutically acceptable carriers for use in the treatment of confirmed or suspected traumatic brain injury (TBI) in a subject, characterised in that the composition is suitable for intranasal, buccal and/or sublingual application. The invention therefore relates to nose drop, a nasal spray or under-the-tongue drops in the form of a liquid composition for transmucosal administration via nasal or buccal mucous membranes.

In embodiments, the invention relates to a pharmaceutical composition comprising a compound Alpha-1062 or salt thereof for use in the treatment of confirmed or suspected traumatic brain injury (TBI) in a subject via transmucosal administration, wherein the composition is an aqueous solution, comprising 2 to 40%, preferably 5 to 15% and more preferably 10% weight per volume (w/v) of the chemical substance.

In embodiments, the invention relates to a pharmaceutical composition comprising a compound Alpha-1062 or salt thereof for use in the treatment of confirmed or suspected traumatic brain injury (TBI) in a subject, wherein the compound Alpha-1062 or salt thereof is present at a concentration of 1 to 200 mg/mL, preferably at a concentration of 5 to 100 mg/mL.

In embodiments, the invention relates to a pharmaceutical composition comprising a compound Alpha-1062 or salt thereof for use in the treatment of confirmed or suspected traumatic brain injury (TBI) in a subject, wherein the compound Alpha-1062 is present from a gluconate salt, preferably at a concentration of 50 to 100 mg/mL.

In embodiments, the invention relates to a pharmaceutical composition comprising a compound Alpha-1062 or salt thereof for use in the treatment of confirmed or suspected traumatic brain injury (TBI) in a subject, wherein the composition is administered at a dose of 0.1 to 200 mg, preferably 1 to 100 mg, more preferably 2 to 40 mg, one to three times daily.

In embodiments, the invention relates to a pharmaceutical composition comprising a compound Alpha-1062 or salt thereof for use in the treatment of confirmed or suspected traumatic brain injury (TBI) in a subject, wherein the composition is administered intranasally as a 2 to 40% weight per volume (w/v) solution of Alpha-1062, at an amount of 20 to 100 microliters, preferably in a single spray event, preferably one to three times daily, preferably over a period of five to 30 days after the TBI.

In embodiments, the composition is administered to the oral cavity as a 2 to 40% weight per volume (w/v) solution of Alpha-1062, at an amount of 20 to 100 microliters, preferably in a single spray event, preferably one to three times daily, preferably over a period of five to 30 days after the TBI.

In embodiments, the invention relates to a pharmaceutical composition comprising a compound Alpha-1062 or salt thereof for use in the treatment of confirmed or suspected traumatic brain injury (TBI) in a subject, wherein the

composition is administered intranasally, buccally or sublingually, as an about 10% weight per volume (w/v) solution at an amount of 20-100 microliters, e.g. about 50 microliters, in 1-3 administration events, preferably in a single administration event, twice daily, preferably over multiple days.

In embodiments, the pharmaceutical composition is an aqueous solution, comprising 2 to 20% weight per volume (w/v), preferably 5 to 15% weight per volume (w/v), more preferably 10% weight per volume (w/v) of the chemical substance.

To be suitable for transmucosal delivery in the oral or nasal cavity, in some embodiments the composition is formulated as high-concentration aqueous salt solutions, or as emulsions, or as selfmicroemulsifying drug delivery systems (SMEDDs) or as micronized powder formulations.

In embodiments, the dosage regimes described herein are employed using a multi-use dispenser, as described herein. Features disclosed with respect to the dispenser are also considered disclosed with respect to the dosage regime, and vice versa.

In embodiments, the concentrations of Alpha-1062 or salt thereof employed, preferably in the aforementioned dosage regimes, are preferably in solution or as emulsions, at about 1%, 2, 3, 4, 5, 6, 7, 8, 9, 10, 11, 12, 13, 14, 15, 16, 17, 18, 19, 20, 25, 30, 35, 40, 45, or50wt%, or more. Dosage regimes disclosing administration of about 10 wt%, preferably 10% solutions or emulsions, may be employed alternatively at any of the afore-mentioned concentrations, or values similar thereto. Concentration ranges constructed form any given of the afore-mentioned values are also contemplated.

In some embodiments, it is intended to use Alpha-1062 or salts thereof in manner that enables distribution of the substance in a patient after administration at a brain-to-blood concentration ratio of more than 5, preferably more than 10, more preferably between 15 and 25.

The invention also relates to a pharmaceutical composition comprising a compound Alpha-1062 or salt thereof for use in the treatment of confirmed or suspected traumatic brain injury (TBI) in a subject, wherein the composition is administered within 24 hours of injury.

In another embodiment the composition is administered within 1 hour of injury.

In embodiments, the administration subsequent to TBI with at little delay as possible, is preferred, such as within 1 week, 6 days, 5 days, 4 days, 3 days, 2 days, or within 1 day of TBI. In embodiments, the administration is carried out within 24 hours, 12 hours, 8 hours, 6, 5, 4, 3, 2 or within 1 hour of TBI.

In embodiments, the administration subsequent to TBI is carried out within 1, 2 or 3 weeks, within 1 month or within 1 year of TBI. In embodiments, the administration is carried out within 48, 36, 24 months, 12 months, 6 months, 5, 4, 3, 2 or within 1 month of TBI.

The dosage regimes as described herein according to some embodiments represent novel and surprisingly beneficial developments in comparison to the prior art with respect to effective galantamine treatment. The biological and medical effect of galantamine has never previously been tested with regard to the potential effect generated by administration at high doses. Many patients in need of galantamine treatment have not been able to be treated due to the significant side effects that occur with regular doses of galantamine. In order to obtain meaningful levels of galantamine in the brain of subjects, the prior art teaches high but also highly intolerable doses. Because only a small fraction of orally or intranasally administered galantamine drug reaches the brain, the dose required to show an effect during treatment of brain disease is often intolerably high due to the large amount of galantamine in other tissues of the body, thereby causing unwanted side effects.

Embodiments and features of the invention described with respect to the pharmaceutical composition, the substance and salts thereof, the multi-use dispenser, and various methods described herein, are considered to be disclosed with respect to each and every other aspect of the disclosure, such that features characterizing the methods, or dispenser, may be employed to characterize the composition, or the substance and vice-versa. The various aspects of the invention are unified by, benefit from, are based on and/or are linked by the common and surprising finding of the beneficial and curative effect of the use of Alpha-1062 in the treatment of TBI, especially when administered via the transmucosal route.

## DETAILED DESCRIPTION OF THE INVENTION

The present invention relates to pharmaceutical compositions comprising a compound Alpha-1062 or salt thereof, or to the compound Alpha-1062 or salt thereof, for use in the treatment of confirmed or suspected traumatic brain injury (TBI) in a subject. The invention also relates to a single- or multi-use dispenser configured for intranasal or transmucosal administration of the compounds or compositions of the invention as well as to various treatment methods of TBI comprising administering a composition comprising Alpha-1062 or salt thereof.

The preferred molecule of the present invention is Alpha-1062, also known formerly as GLN-1062 or Memogain®. The form employed in the examples of the invention, and in one preferred embodiment of the invention, is the gluconic acid salt of Alpha-1062 (Alpha-1062 gluconate). For the sake of completeness, the compound Alpha-1062 is a galantamine pro-drug, which exhibits no or negligible activity as a cholinesterase inhibitor or nicotinic modulator prior to

cleavage. Upon esterase cleavage galantamine is released.

The term "active agent" or "active pharmaceutical ingredient" (API) may however be used for Alpha-1062, as in some embodiments it is the preferred compound of the present invention. In other embodiments, the compound galantamine may also be considered an active agent or relevant drug molecule.

The chemical name (IUPAC) of Alpha-1062 is (4aS,6R,8aS)- 5,6,9,10,11,12-hexahydro-3-methoxy-11-methyl-4aH-[1]benzofuro[3a,3,2-ef][2]benzazepin-6-ol benzoate.

Molecular formula of free base: $C_{24}H_{25}NO_4$; Molecular formula of gluconic acid: $C_6H_{12}O_7$; Molecular weight of free base: 391.47 g/mol; Molecular weight of Alpha-1062 gluconate: 587.61 g/mol; Conversion factor: 1 mg base = 1.501 mg salt.

Chemical Structure of Alpha-1062:

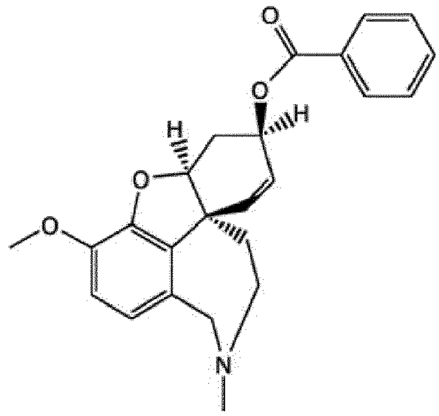

The chemical structure of the Alpha-1062 gluconate is as follows:

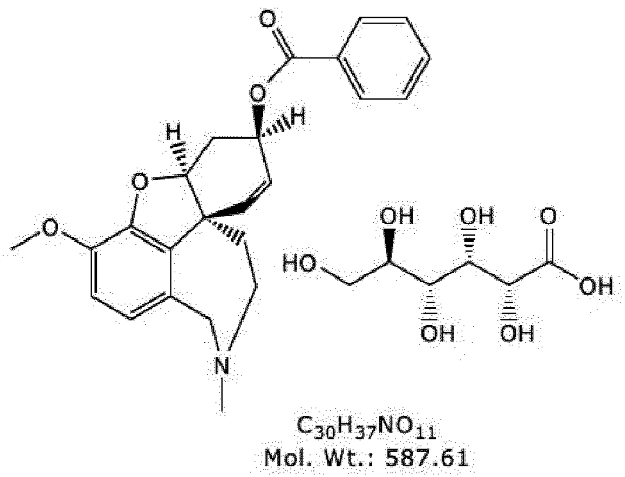

$C_{30}H_{37}NO_{11}$
Mol. Wt.: 587.61

Galantamine has a formula of $C_{17}H_{21}NO_3$, and a molar mass of 287.359 g·mol$^{-1}$, and a structure of:

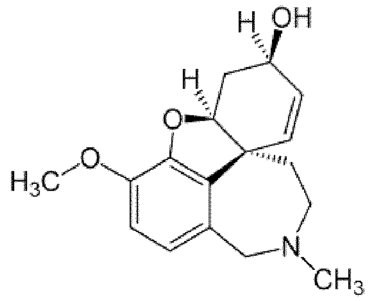

As used herein, the term "self-preserving" is a description of an anti-microbial property of a compound, namely of Alpha-1062 or salt thereof, or a composition comprising such an agent, that does not require the presence of an additional antimicrobial preserving agent.

In preferred embodiments, a self-preserving liquid composition maintains the absence of, has low or negligible numbers, or relatively slow rates of growth, of viable microbes present in the composition, or reduces the numbers of viable microbes in the composition. In some embodiments, the property "self-preserving" indicates that the rate of microbial expansion (cell growth or division) in the composition over time is lower than in the absence of the relevant compound (Alpha-1062). As used herein, a "self-preserving" liquid composition will therefore exhibit lower numbers of viable microbes in comparison to a composition without such "self-preserving" properties. In some embodiments, a "self-preserving" composition comprises no additional anti-microbial preservatives and shows no or negligible increase in the numbers of viable microbes in the composition for an extended period, preferably at least 14 days, or for at least 28 days.

As used herein, the term "anti-microbial" describes the property of a compound or composition to reduce numbers of viable microbes or show no or a negligible increase in the numbers of viable microbes in the composition for at least 14 days, or for at least 28 days. In some embodiments of the invention, pathogenic fungi, gram-negative and/or gram-positive bacteria can be killed or inhibited in growth by Alpha-1062 or salts thereof. In some embodiments, the term "anti-microbial" is defined according to the guidelines set out for preservatives in the USP 51 test. The term may therefore depend on the type of microbe and duration of test. The concentration of an added antimicrobial preservative is usually kept at a minimum, or avoided completely, especially if the active ingredients of the formulation possess an intrinsic antimicrobial activity, as is the case with Alpha-1062 or salts thereof. Antimicrobial effectiveness, whether inherent in the product or whether produced because of the addition of an antimicrobial preservative, must commonly be demonstrated for multiple-dose topical and oral dosage forms and for other dosage forms such as ophthalmic, optic, nasal, irrigation, and dialysis fluids. As described herein, the agent Alpha-1062 exhibits anti-microbial preservative properties, inherent in the molecule, and therefore requires little, or no additional usage of additional preservatives in the composition of the invention.

"Traumatic Brain Injury" (TBI) is a disruption in the normal function of the brain that can be caused by a blow, bump or jolt to the head, the head suddenly and violently hitting an object or when an object pierces the skull and enters brain tissue. Observing one of the following clinical signs constitutes alteration in the normal brain function: Loss of or decreased consciousness, loss of memory for events before or after the event (amnesia), focal neurological deficits such as muscle weakness, loss of vision, change in speech, alteration in mental state such as disorientation, slow thinking or difficulty concentrating

Symptoms of a TBI can be mild, moderate, or severe, depending on the extent of damage to the brain. Mild cases may result in a brief change in mental state or consciousness. Severe cases may result in extended periods of unconsciousness, coma, or even death.

Symptoms of TBI can vary greatly depending on the severity of the head injury and may comprise vomiting, lethargy, headache, confusion, paralysis, coma, unconsciousness, dilated pupils, visual disturbances (blurred or double vision, intolerance to bright light, loss of eye movements, blindness) dizziness and imbalance, difficulty swallowing, numbness or tingling sensation, drooping eyelids or weak face, loss of bowel or bladder control.

Short and long term symptoms of TBI can further comprise one or more of the symptoms selected from the group comprising dizziness, balance problems, headaches, nausea, vomiting, light sensitivity, impaired memory, sleep abnormalities, impaired concentration and impaired vision, weakness in arms and legs, balance and coordination problems, severe or increasing headaches, impaired sensory perception, impaired cognitive skills, impaired memory, impaired communication and learning, personality changes, behavior abnormalities, impaired vision and hearing, paralysis, coma, loss of consciousness, dilated pupils, loss of cerebrospinal fluid from the ears or nose, loss of bowel and/or bladder control, breathing problems, slow pulse, breathing problems, slow breathing rate with an increase in blood pressure, droopy eyelid or facial weakness, hematoma, contusion, intracerebral hemorrhage, subarachnoid hemorrhage, diffuse injuries, diffuse axonal injury, ischemia, primary brain injury and/or secondary brain injury.

As used herein, "primary brain injuries", i.e. such as skull fractures, localized injuries or diffuse axonal injury, occur at the time of the initial brain injury, i.e. a TBI event, and cannot be prevented, but only addressed by subsequent treatment. One goal of the treatment of an initial brain injury is to prevent or reduce further, or "secondary brain injuries". The term "secondary brain injury" typically refers to the changes that evolve after the initial injury, usually within minutes, hours, days or sometimes even months. It comprises an entire series of events or stages of cellular, chemical, tissue, or blood vessel changes in the brain that contribute to further destruction of brain tissue.

TBI can cause "mass lesions," an area of localized injury such as bruises and bruises that increase pressure in the brain. Secondary injuries cause by TBI or accompanying TBI can comprise one or more of the following injuries: hematoma, (brain) bruises, intracerebral hemorrhage, subarachnoid hemorrhage, diffuse Injuries, diffuse axon injury, ischemia and/or skull fractures.

In embodiments of the invention "hematoma" refers to a blood clot in the brain or on its surface that can appear anywhere in the brain. An epidural hematoma is a collection of blood between the dura mater (the protective covering of the brain) and the inside of the skull. A subdural hematoma is a collection of blood between the dura mater and the arachnoid layer that is located on the surface of the brain.

In embodiments of the invention "bruise" or "brain bruise" may refer to a bruise of the brain tissue that is pathologically comparable to bruises on other parts of the body. A brain bruise consists of areas of injured or swollen brain mixed with blood that has leaked from arteries, veins, or capillaries. The most common bruises are at the base of the front parts of the brain, but they can appear anywhere.

In embodiments of the invention "intracerebral hemorrhage" (ICH) refers to hemorrhages in the brain tissue that can be related to other brain injuries, especially bruises. Depending on the size and location of the bleeding it could be surgically removed.

In embodiments of the invention "subarachnoid hemorrhage" (SAH) is caused by bleeding into the subarachnoid space. It appears as diffuse blood that spreads thinly over the surface of the brain and is common after TBI. Most cases of SAH related to head trauma are mild. Hydrocephalus can result from severe traumatic SAH.

In embodiments of the invention "diffuse Injuries" describes microscopic changes that do not appear on CT scans and are scattered throughout the brain, which can occur as a result of TBI. This category of injury, known as diffuse brain injury, can occur with or without an associated mass lesion.

In embodiments of the invention "diffuse axon injury" refers to impaired function and gradual loss of axons. These long extensions of nerve cells enable them to communicate with one another. If enough axons are damaged in this way, the ability of nerve cells to communicate with one another and integrate their function can be lost or severely impaired, which can lead to severe disabilities for the patient.

In embodiments of the invention "ischemia" describes another type of diffuse injury, or an insufficient blood supply to certain parts of the brain. A high percentage of TBI patients experience a decrease in blood supply to very low levels. This is crucial because a brain that has just suffered a traumatic injury is particularly sensitive to mild reductions in blood flow. Changes in blood pressure in the first few days after a head injury can also have an adverse effect. Ischemia is also considered to be a major cause of secondary brain injury.

In embodiments of the invention "skull fractures" refer to linear skull fractures or simple fractures in the skull that can accompany TBI.

Potential forces strong enough to cause a fractured skull can damage the brain below. Skull fractures can be alarming when discovered during a patient exam. Fractures at the base of the skull are problematic because they can injure nerves, arteries, or other structures. If the fracture extends into the sinuses, a leakage of liquor (liquor) from the nose or ears can occur. Depressed skull fractures, in which part of the bone presses on or into the brain, can also occur.

A "pro-drug" in general describes a medication or compound that, after administration, is metabolized within the body into a pharmacologically active drug. A prodrug can be administered instead of its corresponding drug compound to improve the absorption, distribution, metabolization, and/or excretion of the corresponding drug. The use of prodrugs with improved bioavailability is especially advantageous, if the corresponding drug is poorly absorbed, e.g., through the gastrointestinal route. The administration of a prodrug may reduce adverse or undesired side-effects of a corresponding drug and/or improve the drugs bioavailability and/or absorption.

"Pro-drugs" are typically *per se* therapeutically inactive agents that are predictably transformed in specific locations in the body to active metabolites. In this sense, pro-drugs are inactive precursors of parent drugs that undergo transformation into active agents *in vivo* by enzymatic cleavage or chemical spontaneous process(es) in a predictable fashion. In the pro-drugs according to some embodiments discussed herein, there exists preferably a covalent ester linkage between the parent drug and the selected transport pro-moiety, and upon cleavage of this ester bond, ideally in the target organ brain, the inactive pro-drug releases the active parent drug at or close to its target sites in the CNS.

Herein, a "pro-drug" may refer to Alpha-1062 or salts thereof, without being limited to. In some embodiments of the invention the administration of the pro-drug Alpha-1062 or salts thereof is intended to reduce or prevent (partially or entirely) galantamine-related adverse effects and/or improve the transmucosal absorption of galantamine compounds.

A goal of some embodiments according to the present invention is therefore to present novel CNS therapeutics

for the treatment of TBI having optimal brain bioavailability due to being formulated as lipophilic pro-drugs and administered via transmucosal absorption pathways in the oral or nasal cavity.

The Alpha-1062 pro-drugs disclosed herein are passively transported through the blood-brain barrier (BBB) into the brain. These Alpha-1062 pro-drugs are pharmacologically inactive and hence do not produce any significant GI or other side effects, as long as they remain un-cleaved in the particular tissue. After enzymatic cleavage, from each molecule of pro-drug one molecule of parent drug is formed, thereby producing the full pharmacological effect of the drug. If cleavage is preferentially in the brain, due to enhanced distribution into this organ, and the availability of suitable endogenous enzyme(s) therein, a persistently higher concentration of drug at the target sites in the CNS and consequently larger medically beneficial effects are achieved. Preferential transport to the target organ brain is further optimized in a surprising and beneficial manner by transmucosal routes of administration in the oral or nasal cavity.

In summary, these features of the formulations of Alpha-1062 or salts thereof described for some embodiments herein, foster delivery to the brain of more persistent and higher concentrations of drug than can be achieved by oral administration in form of tablets of the unmodified drug. The improved distribution of the drug to the brain dramatically reduces all locally produced side effects in the GI tract, thereby permitting to immediately apply an efficacious dose of the drug to its CNS-located target molecules, e.g. nicotinic receptors and cholinesterases.

As the "blood-brain barrier" (BBB), located at the level of the brain capillaries, is the major barrier to the passage of drugs from the blood compartment to the brain, initial focus on optimizing penetration of the pro-drugs through the BBB yielded promising results. The brain microvessel endothelial cells forming the BBB have as typical morphological characteristics tight junctions between cells, absence of fenestrations and diminished pinocytotic activity. A variety of enzymes further contributes to the restrictive nature of the BBB. The ability of drugs to cross the BBB mostly depends on their physicochemical properties, such as their lipophilicity. Consequently, the compounds considered in the present disclosure all are pro-drugs with improved lipophilicity, in comparison to their parent compounds.

The "BBCR" is to be understood as the brain-to-blood concentration ratio after transport equilibrium via the BBB has been achieved.

The term "transmucosal administration" relates to the entering of a pharmaceutical agent through, or across, a mucous membrane. The transmucosal routes of administration of the present invention are defined as intranasal, buccal and/or sublingual.

"Nasal or intranasal administration" relates to any form of application of the prodrug or pharmaceutical composition thereof to the nasal cavity. The nasal cavity is covered by a thin mucosa which is well vascularised. Therefore, a drug molecule can be transferred quickly across the single epithelial cell layer without first-pass hepatic and intestinal metabolism. Intranasal administration is therefore used as an alternative to oral administration of for example tablets and capsules, which lead to extensive degradation in the gut and/or liver.

"Buccal administration" relates to any form of application that leads to absorption across the buccal mucosa, preferably pertaining to adsorption at the inside of the cheek, the surface of a tooth, or the gum beside the cheek.

"Sublingual administration" refers to administration under the tongue, whereby the chemical comes in contact with the mucous membrane beneath the tongue and diffuses through it.

Rapid absorption in the oral cavity is best achieved by sublingual administration, as the mucosal thickness in this area is lower than in other buccal areas, in addition to being significantly less keratinized (Shojaei A (1998) Buccal mucosa as a route for systemic drug delivery: a review. J Pharm Pharmaceut Sci 1: 15-30). Fast dissolving sublingual formulations according to some embodiments of the invention, such as rapidly degrading tablets or liquid-filled capsules, can additionally help reducing enzymatic degradation of pro-drug in saliva. The nasal cavity also provides a promising starting point for alternative administration regimes, with its large surface area, high vasculature and low enzymatic environment. Intranasal delivery according to some embodiments of the invention is capable of providing a similarly high level of bioavailability as intravenous administration with the advantages of non-invasiveness, ease of self-administration, patient comfort and patient compliance in comparison to the latter. These advantages may have been known generally by practitioners of the art; however, significant hurdles remain for developing such application routes. For chronic systemic delivery, the problems of epithelial damage and toxicity need to be solved, and that for sufficient bioavailability high concentrations of drug in small volumes of vehicle are provided. This requires first selection of suitable chemical compounds that enable the required formulations and concentrations, in addition to finding appropriate methods for administration and finally developing preferred salts and/or solutions thereof that allow optimal administration of effective substance to the brain.

Pharmaceutical compositions suitable for buccal and/or sub-lingual administration may comprise in some embodiments additional "pharmaceutically acceptable carriers", for example a buccal dosage unit may comprise the active agent to be administered in addition to a polymeric carrier that bioerodes and provides for delivery of the active agent over a predetermined time period, and, preferably, a lubricant, such as magnesium stearate. Additional carrier agents are known to one in the art. This active agent can be physically compounded with materials of some or all of classes of ingredients that function as pH controls, preservative agents, viscosity control agents, absorption enhancers, stabilizing agents, solvents, and carrier vehicles. Such agents may in some embodiments of the invention be present in either solid

or liquid forms of the pharmaceutical composition.

A "self-microemulsifying drug delivery system" (SMEDDS) may be present in said pharmaceutical composition in some embodiments, meaning a drug delivery system that uses a microemulsion achieved by chemical rather than mechanical means. That is, by an intrinsic property of the drug formulation, rather than by special mixing and handling. It employs the familiar effect displayed by anethole in many anise-flavored liquors. Microemulsions have significant potential for use in drug delivery, and SMEDDS (including so-called "U-type" microemulsions) are the best of these systems identified to date. SMEDDS are of particular value in increasing the absorption of lipophilic drugs taken by mouth. SMEDDS in may include in a non-limiting manner include formulations of the drugs anethole trithione, oridonin, curcumin, vinpocetine, tacrolimus, berberine hydrochloride, nobiletin and/or piroxicam. Other emulsions are generated by mechanical means, such as mixing, sonicating, vortexing or homogenizing. The first drug marketed as a SMEDD was cyclosporin, and it had significantly improved bioavailability compared with the conventional solution. SMEDDS offer numerous advantages: spontaneous formation, ease of manufacture, thermodynamic stability, and improved solubilization of bioactive materials. Improved solubility contributes to faster release rates and greater bioavailability. For many drugs taken by mouth, faster release rates improve the drug acceptance by consumers. Greater bioavailability means that less drug need be used; this may lower cost and does lower the stomach irritation and toxicity of drugs taken by mouth. For oral use according to some embodiments, SMEDDS may be formulated as liquids, and are applicable for transmucosal administration.

The pharmaceutical composition may in some embodiments include one or more pharmaceutically acceptable carriers, or excipients. The term "excipient" means a pharmacologically inactive component such as a diluent, disintegrant, carrier, and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient. The excipients are described herein in some embodiments according to "wt%", or "percentage by weight".

"Administration" or "treatment," as it applies to an animal, human, experimental subject, cell, tissue, organ, or biological fluid, refers to contact of a pharmaceutical, therapeutic, diagnostic agent, compound, or composition to the animal, human, subject, cell, tissue, organ, or biological fluid. "Administration" and "treatment" can refer in some embodiments, e.g., to therapeutic, placebo, pharmacokinetic, diagnostic, research, and experimental methods. "Treatment," as it applies to a human, veterinary, or research subject, refers to therapeutic treatment, prophylactic or preventative measures, to research and diagnostic applications. As used herein, "administer" or "administration" refers to the delivery of the drug or agent of the present invention or a pharmaceutical composition thereof to an organism for the purpose of prevention or treatment of a brain disease associated with cognitive impairment. Suitable routes of administration may include, without limitation, oral, rectal, transmucosal or intestinal administration or intramuscular, subcutaneous, intramedullary, intrathecal, direct intraventricular, intravenous, intravitreal, intraperitoneal, intranasal, sublingual, buccal or intraocular injections. The preferred route of administration is transmucosal.

In some embodiments, the liquid formulations are configured for any one or more of the above administration modes. A skilled person is aware of technical means employed in configuring compositions for specific modes of administration. For example, a composition configured for nasal administration may be formulated, packaged, or prepared in a different manner from compositions prepared for oral administration.

Pharmaceutical compositions of the present invention may be manufactured in some embodiments by processes well known in the art, e.g., by means of conventional mixing, dissolving, granulating, dragee-making, levigating, emulsifying, encapsulating, entrapping, dissolving or lyophilizing processes. Pharmaceutical compositions for use in accordance with the present invention may in some embodiments be formulated in conventional manner using one or more physiologically acceptable carriers including excipients and auxiliaries that facilitate processing of crystals or other forms of the present invention into preparations that can be used pharmaceutically. Proper formulation is dependent upon the route of administration chosen.

In some embodiments for injection or transmucosal administration, a compound of the present invention or a pharmaceutical composition thereof may be formulated in aqueous solutions, preferably in physiologically compatible buffers such as Hanks' solution, Ringer's solution, or physiological saline buffer. For transmucosal administration according to some embodiments, penetrants appropriate to the barrier to be permeated are used in the formulation. Such penetrants are generally known in the art.

In some embodiments for oral administration, a compound of the present invention or a pharmaceutical composition thereof can be formulated by combining a compound of the present invention with pharmaceutically acceptable carriers well known in the art. Such carriers enable compounds of the present invention in some embodiments to be formulated as tablets, pills, lozenges, dragees, capsules, liquids, gels, syrups, slurries, suspensions and the like, for oral ingestion by a patient. Pharmaceutical preparations for oral use can be made in some embodiments using a solid excipient, optionally grinding the resulting mixture, and processing the mixture of granules, after adding other suitable auxiliaries if desired, to obtain tablets or dragee cores. Useful excipients are, in particular, fillers such as sugars, starch and other materials. If desired, disintegrating agents may be added.

"Effective amount" also relates to an amount of the prodrug substance or pharmaceutical composition thereof, sufficient to allow or facilitate the amelioration and/or diagnosis of a symptom or sign of a disorder, condition, or pathological state. The term "effective amount" or "therapeutically effective amount" used interchangeably, is defined to mean the amount or quantity of the compound (e.g. Alpha-1062 or salts thereof), which is sufficient to elicit an appreciable biological response when administered to the patient in some embodiments. It will be appreciated that the precise therapeutic dose will depend on the age and condition of the patient, nature of the condition to be treated and will be at the ultimate discretion of the attendant physician. The election of dose in this case will relate to both therapeutic effect on the treatment of neurological disease, in addition to an effective amount with respect to the anti-microbial effect. Both amounts may be assessed and determined by a skilled person.

The invention encompasses in some embodiments administration of an effective amount of chemical substance as described herein to a patient or subject in need thereof. "Effective amount" or "therapeutically effective amount" means an amount sufficient to ameliorate a symptom or sign of a disorder or physiological condition or an amount sufficient to permit or facilitate a diagnosis of the disorder or physiological condition. An effective amount for a particular patient or veterinary subject may vary depending on factors such as the condition being treated, the overall health of the patient, the method route and dose of administration and the severity of side effects. An effective amount can be the maximal dose or dosing protocol that avoids significant side effects or toxic effects. The effect will result in an improvement of a diagnostic measure, parameter, or detectable signal by at least 5%, usually by at least 10%, more usually at least 20%, most usually at least 30%, preferably at least 40%, more preferably at least 50%, most preferably at least 60%, ideally at least 70%, more ideally at least 80%, and most ideally at least 90%, where 100% is defined as the diagnostic parameter shown by a normal subject.

Herein, the terms "subject", "patient" or "individual" may be used interchangeably. In preferred embodiments of the invention a subject may be an animal, a mammal or a human, even more preferably a human.

As used herein, the term "liquid" refers to its common meaning, including compositions with nearly incompressible fluid that conforms to the shape of its container but retains a (nearly) constant volume independent of pressure.

As used herein "pharmaceutical compositions in liquid form" are liquids comprising one or more pharmaceutically active agents, suitable for administration to a subject, preferably a mammal, more preferably human subject. Liquid dosage forms are typically pharmaceutical products which involve a mixture of drug components and nondrug components (excipients). Liquid dosage forms are prepared: a) by dissolving the active drug substance in an aqueous or non-aqueous solvent (e.g. water, glycerin, ether, alcohol), or b) by suspending the drug in appropriate medium, or c) by incorporating the drug substance into an oil or water phase, such as suspensions, emulsions, syrups or elixirs. The solutions described herein are characterized by good solubility in water, as is evident for the salts of Alpha-1062.

"Emulsions" may also be produced and employed for transmucosal administration, as disclosed in the art. An "emulsion" is a mixture of two or more liquids that are normally immiscible (unmixable or unblendable). Emulsions are part of a more general class of two-phase systems of matter called colloids. Although the terms colloid and emulsion are sometimes used interchangeably, emulsion should be used when both phases, dispersed and continuous, are liquids. In an emulsion, one liquid (the dispersed phase) is dispersed in the other (the continuous phase). Examples of emulsions include creams, ointments, liniments (balms), pastes, films, or liquids, depending mostly on their oil-to-water ratios, other additives, and their intended route of administration. Many are topical dosage forms and may be used on the surface of the skin, transdermally, transmucosally, ophthalmically, rectally or vaginally. A highly liquid emulsion may also be used orally, intranasally or may be injected in some cases.

Pharmaceutical compositions of the present invention may be manufactured by processes well known in the art, e.g., by means of conventional mixing, dissolving, granulating, levigating, emulsifying, encapsulating, entrapping, dissolving or lyophilizing processes. Pharmaceutical compositions for use in accordance with the present invention may be formulated in conventional manner using one or more physiologically acceptable carriers including excipients and auxiliaries that facilitate processing the API into preparations that can be used pharmaceutically. Proper formulation is dependent upon the route of administration chosen.

For injection or transmucosal administration, the API or a pharmaceutical composition thereof may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For oral administration, the API the present invention or a pharmaceutical composition thereof can in some embodiments be formulated by combining the API with pharmaceutically acceptable carriers well known in the art. Such carriers enable substances, such as in crystal form, of the present invention to be formulated as tablets, pills, lozenges, capsules, liquids, gels, syrups, slurries, suspensions, solutions and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth,

methyl cellulose, hydroxypropylmethyl-cellulose, sodium carboxymethylcellulose, and/or polyvinyl-pyrrolidone (PVP). If desired, disintegrating agents may be added, such as cross-linked polyvinyl pyrrolidone, agar, or alginic acid. A salt such as sodium alginate may also be used.

A composition of the present invention may in some embodiments also be formulated for parenteral administration, e.g., by bolus injection or continuous infusion. Formulations for injection may be presented in unit dosage form, e.g., in ampoules or in multi-dose containers, with an added preservative. The compositions may take such forms as suspensions, solutions, or emulsions in oily or aqueous vehicles, and may contain formulating materials such as suspending, stabilizing, and/or dispersing agents.

Various liquid compositions comprising Alpha-1062 or salt thereof, in addition to solutions, are contemplated, such as emulsions, suspensions, and the like. In one embodiment, the liquid pharmaceutical composition comprising Alpha-1062 or salt thereof comprises a sufficient amount or volume of composition for multiple administration events (doses) to a subject. The invention therefore also relates to pharmaceutical compositions comprising Alpha-1062 or salt thereof in a solution configured for multi-dose administration to a subject in need thereof.

Pharmaceutical compositions for parenteral (any route that is not gastrointestinal) administration include aqueous solutions of a water-soluble form of the API. Additionally, suspensions of drug or pro-drug of the present invention or pharmaceutical compositions thereof may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances that increase the viscosity of the suspension, such as sodium carboxymethyl cellulose, sorbitol, or dextran. Optionally, the suspension may also contain suitable stabilizers and/or agents that increase the solubility of the compounds of the present invention or a pharmaceutical composition thereof to allow for the preparation of highly concentrated solutions.

In some embodiments, the composition comprising active ingredient may be in powder form. For example, the powder may be prepared for constitution with a suitable vehicle, e.g., sterile, pyrogen-free water, before use. In some embodiments, the powder formulation is for administration to a subject. For example, powder formulations, such as micronized powder formulations, may be administered intranasally, or via other methods, such as via transmucosal administration, by spraying or otherwise applying the powder formulation to a subject, preferably to a mucosal surface of a subject.

The antimicrobial properties of Alpha-1062 as described herein are beneficial for not only liquid formulations, such as solutions, suspensions and emulsions, but also to solid or powder formulations. Powder formulations suitable for transmucosal administration also benefit from the antimicrobial properties of Alpha-1062 and the absence of additional antimicrobial preservatives, made possible by the finding that Alpha-1062 is itself antimicrobial. Similarly, solid compositions may also benefit from the antimicrobial properties of Alpha-1062 and the absence of additional antimicrobial preservatives, leading potentially to reduced microbial contamination and longer storage times for any given Alpha-1062 composition.

Determination of a therapeutically effective amount and suitable mode of administration is within the capability of those skilled in the art, especially in light of the detailed disclosure provided herein. The amount of a composition administered will, of course, be dependent on the subject being treated, the severity of the affliction, the manner of administration, the judgment of the prescribing physician, etc.

The compositions may, if desired, be presented in a pack or dispenser device, such as an EMA and/or FDA approved kit, which may contain dosage forms containing the active ingredient. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Such notice, for example, may be of the labelling approved by the European Medicines Agency (EMA) and/or U.S. Food and Drug Administration (FDA) for prescription drugs or of an approved product insert.

Compositions including a compound of the present invention formulated in a compatible pharmaceutical carrier may also be prepared, placed in an appropriate container, and labelled for treatment of an indicated condition.

"Multi-use dispensers" are known to those skilled in the art and may come in the form of any device suitable for multiple doses or multiple applications of the compound. In other words, herein it is preferred that a multi-use device does not require opening or re-filling between multiple administration events and is typically stable, with sufficient API stability and low microbial loads, over extended periods. In some embodiments, the dispenser may be suitable for administration of any given liquid formulation, preferably a solution, although emulsions and suspensions, and the like, are contemplated.

"Liquid formulations" are known to the skilled person and are common in pediatric or geriatric patient populations. A liquid formulation typically requires a stable, dissolved, or suspended form of the compound that meets release, bioavailability, and taste/irritation requirements. Both immediate and sustained release liquid products are contemplated. Liquid formulation strategies typically include direct incorporation of an active drug or pro-drug in a dissolved or suspended or emulsified state. Alternative states include incorporation of an active drug or pro-drug in the form of a suspended

drug-ion exchange resin complex, or incorporation of drug or pro-drug in the form of a dissolved or suspended drug-cyclodextrin inclusion complex, or in an emulsified state.

"Dispensers" configured for transmucosal administration are also known in the art, as are transmucosal administration routes, which relate preferably to oral, nasal, vaginal, and urethral modes. The transmucosal membranes are relatively permeable, have a rich blood flow and hence allow the rapid uptake of a drug into systemic circulation to avoid first pass metabolism. The oral transmucosal delivery preferably relates to the buccal and sublingual routes. This route of drug delivery offers a number of benefits over other drug delivery approaches and allows drugs to circumvent some of the body's natural "defense mechanisms" like first pass metabolism, the harsh stomach environment, and potentially metabolism in the intestines due to exposure to microbial populations present in the intestines. By way of example, several approaches have been used like drug delivery through the nasal route by using sprays, pumps and gels while the mucoadhesive, quick dissolve tablets and solid lozenge formulations are suitable for the oral mucosal route, also, vaginal or urethral routes can be explored using mucoadhesive suppositories, in-situ gel and foam formulations.

Preferred dispensers or multi-use dose devices according to the invention relate to any of the above-mentioned multi-use devices. In some embodiments, the dispensers available for example from Nemera (La Verpilliere, France) or Aptar Pharma (Illinois, USA) are preferred. For example, Nemera provides Advancia® nasal spray dispensers, which are high-performing pumps with excellent dose consistency and prime retention, anti-clogging actuators, no metal contacting the formulation and hygienic anti-actuation snap-on overcaps. As a further example, Aptar Pharma's nasal pump technology removes the need for drug manufacturers to add preservatives to nasal spray formulations. The Advanced Preservative Free (APF) systems use a tip-seal and filter technology to prevent contamination of the formulation. A spring-loaded tip seal mechanism is employed with a filter membrane in the ventilation channel. The Nemera and Aptar technology, amongst others, offers a number of benefits, such as a metal-free fluid path thereby preventing the oxidization of the formulation, a preservative-free system and therefore an anti-microbial dispenser employing a purely mechanical barrier.

According to Advancia (Nemera) technology, the intake of air into the dispenser takes place via a venting system with a silicone membrane. This technology has a continuous barrier of homogenous material which allows air to diffuse through the silicone, which acts as a permeable membrane. Consequently, the continuous barrier ensures the microbial integrity of the drug. The venting system filters the intake of air using a very fine membrane manufactured from silicone polymer. The silicone membrane is a solid, non-porous material. The membrane is homogenous and does not contain any holes. The membrane's intermolecular distance is of the order of nanometers - allowing the passage of air through the membrane but completely preventing the passage of any liquid or solid particle, including bacteria, due to the silicone membrane structure. The function of the silicone membrane can be compared to an inflated balloon. The balloon is a continuous, waterproof material yet gas slowly passes through the wall of the balloon until the pressures inside and outside reach equilibrium. Moreover, Advancia® PF offers a patented anti-clogging actuator in the upper part of the system, called closing tip. This mechanism ensures that no contamination can enter through the actuator orifice, which therefore provides protection from crystallization and clogging issues, and avoids evaporation to guarantee good prime retention.

Additional technology from Nemera is also contemplated, such as SP270+ and SP370+, which enable very good dose consistency, wide range of dose volumes: 50 $\mu$l to 200 $\mu$l with different actuators, various neck finishes: screw-on, snap-on and crimp-on, and are suitable for liquid solutions and suspensions. For unregulated markets alternative technology from Nemera is contemplated, such as SP27 and SP37. Alternatively, continuous valves for nasal and dermal delivery may be employed, such as Nemera solutions employing pressurized delivery with a neutral propellant agent (nitrogen) or liquid gases, such as CV20 for liquids, Valve 6668 for viscous products or Valve 6685 for powders. Additional Nemera dispensing devices are disclosed, for example, in US9238532, which discloses a tip for dispensing liquid for mounting on a container. A valve includes at least two elements that are mobile in relation to one another, each mobile element including a bearing zone against the other mobile element. One of the mobile elements bears anti-microbial material on or in the immediate proximity of a part of its bearing zone forming a blocking barrier and all surfaces of the dispensing tip in contact with the inside are free of anti-microbial material. As a further example, US9345616 discloses a liquid dispenser device with air intake that reliably guarantees the sterility of the content of a reservoir. The function of taking in air and blocking air-borne micro-organisms s performed, not by an air filter, but by using the gas diffusing properties of certain materials. Thus, a member of a type other than a filter is used, namely a member made of non-porous polymer material. Such a member presents the advantage of passing non-contaminated air in a manner that is more reliable than a filter, which is porous by definition. Other technologies are disclosed in US20150043958, US8827124, US8986266, and US20140231536A1. All cited patent documents are hereby incorporated in their entirety by reference. As further examples, Nemera provides various nasal administration technologies, such as advanced preservative free nasal pumps, classic technology platforms, CPS technology platforms, and VP3, VP6 and VP7 technologies.

As a further example, the nasal spray technology from Aptar provides excellent spray performance, is suitable

for solutions, suspensions and viscous drug formulations including gels, is suitable for gamma-irradiation sterilization, and allows wide range of dose volumes from 45μl to 1,000μl, and offers a wide range of closures, actuators and accessories. As a further example, the CPS technology from Aptar is a highly versatile spray pump designed for the multi-dose delivery of preserved or non-preserved drug formulations for the nasal route. CPS can be used for a range of other applications which include intra-nasal vaccination. Various benefits include wide range of dose volumes: 50 μl to 140 μl, no re-priming needed even when not used regularly, can be sterilized by irradiation. The CPS systems use anti-clogging tip seal technology to minimize crystallization with high-viscosity and high-volatile formulations, proven CPS filter technology avoids entrance of contaminated air into the container, fully validated and tested microbiological integrity, no anti-microbial additives within the pump components, and metal-free fluid pathway components. For example, US9095864 from Aptar discloses a fluid dispenser unit and spray devices, in particular of the nasal type, and preferably applies to sprays incorporating a closure member for closing the dispenser orifice. The device mechanics disclosed therein relates to closing the dispenser orifice completely when the pump is not in use, wherein the closure member can come to co-operate directly with the dispenser orifice by being brought elastically by the return spring of the pump towards its closed position, thereby preventing any germs or bacteria penetrating inside the device between two actuations, and thereby significantly minimizing the risks of contaminating the composition to be dispensed. As another example, US20090294347 discloses a dispensing device for a liquid medium, having a medium reservoir for accommodating the medium, having a dispensing opening for dispensing the medium from the medium reservoir, and having a pressure-equalizing channel which opens out into the medium reservoir and has a microbiologically active filter arrangement inserted therein. All cited patent documents are hereby incorporated in their entirety by reference.

The compositions according to the invention may, if desired, be presented in a pack or dispenser device, such as an FDA approved kit, which may contain dosage forms containing the active ingredient. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Such notice, for example, may be of the labeling approved by the U.S. Food and Drug Administration for prescription drugs or of an approved product insert.

Compositions including a compound of the present invention formulated in a compatible pharmaceutical carrier may also be prepared, placed in an appropriate container, and labeled for treatment of an indicated condition.

Suitable conditions are brain injuries associated with neurological and/or cognitive impairment, preferably those disclosed herein, in particular TBI or other brain traumata or concussions.

**FIGURES**

The invention is further described by the figures. These are not intended to limit the scope of the invention.

*Short description of the figures:*

**Figure 1:** Location and effects of a controlled cortical impact (CCI) on the brain of rats.

**Figure 2:** Functional tests that are carried out on healthy, vehicle treated and Alpha-1062 treated rats to assess sensorimotor and cognitive function after TBI.

**Figure 3:** Results of the sensorimotor function tests where events of the rat's right forelimb foot faults during the walk over a mesh were assessed.

**Figure 4:** Results of the sensorimotor function tests where events of the rat's right hindlimb foot faults during the walk over a mesh were assessed.

**Figure 5:** Results of the sensorimotor tests depicting the results in the scaling of the modified neurological severity score (mNSS).

**Figure 6:** Results of the adhesive removal test for the right forelimb.

**Figure 7:** Results of the Morris water maze test.

**Figure 8:** Further results of the Morris water maze test.

**Figure 9:** Results of the new object recognition test.

**Figure 10:** Weight of the test animals throughout the study (day D0-D35).

*Detailed description of the figures:*

**Figure 1:** The figure illustrates the location and effects of a controlled cortical impact (CCI) on the brain of rats, which are alive but anesthetized during the intervention. This experiment serves as a simulation of TBI and its effects on the brain. A) Anatomy scheme illustrating a rat skull bone structure and the injury that was induced in the left parietal cortex to simulate TBI in a rat (red circle). B) Distribution of the strain caused by the impact of an object on the rat brain, depicted in fringe levels. C) Pathologic overview of the damage caused by an TBI on a rat brain. D) The photograph on the top shows a healthy rat brain (sham). The photograph on the bottom illustrates the size and location of the injury that was induced in the left parietal cortex of a rat's brain to simulate TBI, wherein the rat was sacrificed 7 days after the TBI. E) Haematoxylin and eosin staining of slices of a healthy rat brain (top row) and slices of a rat's brain (bottom row) 7 days after an TBI simulating injury (controlled cortical impact CCI) was induced.

**Figure 2:** The figure illustrates functional tests that are carried out on healthy, vehicle treated and Alpha-1062 treated rats to assess sensorimotor and cognitive function after TBI. Sensimotor function tests. a) The figure depicts tests to assess the modified neurological severity score (mNSS). B) The figure shows a test used for assessing the "foot fault" of an animal. The animal is required to balance over a grid and the frequency of steps missing the grid are counted. This test allows assumptions on the functionality of the parietal cortex of the rat's brain. c) The adhesive removal test consists of applying adhesive tape on each forepaw of the animal and measuring the time-to-contact and the time-to-remove them. This behavior implies correct paw and mouth sensitivity (time-to-contact) and correct dexterity (time-to-remove). Cognitive function tests: a) This picture illustrates the Morris water maze that comprises that a rat is placed in a large circular pool (picture on the left) and is required to find an invisible or visible platform that allows it to escape the water by using various cues. This test is used to study spatial learning and memory and allows assumptions if particular cortical brain regions are healthy or damaged. Usually, the path of the rat through the pool is tracked (picture on the right) and the time spend in the proximity of the platform as well as the time needed until the platform is found by the rat are measured. b) For the new/novel object recognition (NOR) test, after a habituation time in the test space (picture on far left) rats are given the opportunity to explore two identical objects (A) for a predetermined period of time (picture in the middle). After a delay, the animals are then presented with two objects to explore, one of which is the same as in the first exploration trial (A), the other a new object (B) (picture on the right). Depending on the length of the delay between the two trials or depending on the extend of cortical damage, the rats will either explore the novel object for a greater time period, indicating memory for the familiar object, or will explore the novel and familiar objects for the same amount of time, indicating a lack of recall or loss of memory for the familiar object presented during the initial trial.

**Figure 3:** The graph shows the results of the sensorimotor function tests where events of the rat's right forelimb foot faults during the walk over a mesh were assessed. The tests were performed as described in Example 1, wherein rats received either no treatment, vehicle treatment or Alpha-1062 treatment after an TBI event. The graph shows that animals treated with Alpha-1062 after experiencing TBI show less foot faults than animals that received vehicle treatment after TBI. This indicates a significant preservation and constant recovery of locomotor skills in rats that were treated with Alpha-1062 after an TBI event in comparison to rats that received only vehicle treatment. The left bars represent the healthy sham rats that experienced no TBI. Middle bars represent rats that experienced TBI and were subsequently treated with vehicle control treatment. Right bars represent the rats that were treated with Alpha-1062 after experiencing TBI. The detailed treatment regimen and experimental setup can also be found in Example 1. The sham group comprised 8 rats, the vehicle and Alpha-1062 treatment groups comprised each 9 rats.

**Figure 4:** The graph shows the results of the sensorimotor function tests where events of the rat's right hindlimb foot faults during the walk over a mesh were assessed. The tests were performed as described in Example 1, wherein rats received either no treatment, vehicle treatment or Alpha-1062 treatment after an TBI event. The graph shows that animals treated with Alpha-1062 after experiencing TBI show less foot faults than animals that received vehicle treatment after TBI. This indicates a significant preservation and constant recovery of locomotor skills in rats that were treated with Alpha-1062 after an TBI event in comparison to rats that received only vehicle treatment. The left bars represent the healthy sham rats that experienced no TBI. Middle bars represent rats that experienced TBI and were subsequently treated with vehicle control treatment. Right bars represent the rats that were treated with Alpha-1062 after experiencing TBI. The detailed treatment regimen and experimental setup can also be found in Example 1. The sham group comprised 8 rats, the vehicle and Alpha-1062 treatment groups comprised each 9 rats.

**Figure 5:** The graph shows the results of the sensorimotor tests and depicts the results in the scaling of the modified neurological severity score (mNSS). The tests were performed as described in Example 1, wherein rats received either no treatment, vehicle treatment or Alpha-1062 treatment after an TBI event. From day 14 (D14) onwards, the Alpha-1062 treated animals performed statistically significantly better than vehicle treated animals. The left bars represent

the healthy sham rats that experienced no TBI. Middle bars represent rats that experienced TBI and were subsequently treated with vehicle control treatment. Right bars represent the rats that were treated with Alpha-1062 after experiencing TBI. The detailed treatment regimen and experimental setup can also be found in Example 1. The sham group comprised 8 rats, the vehicle and Alpha-1062 treatment groups comprised each 9 rats.

**Figure 6:** The graph shows the results of the adhesive removal test for the right forelimb. The tests were performed as described in Example 1, wherein rats received either no treatment, vehicle treatment or Alpha-1062 treatment after an TBI event. The graph shows that animals that received Alpha-1062 after the TBI event performed from day 7 (D7) on significantly better than the vehicle treated animals. The left bars represent the healthy sham rats that experienced no TBI. Middle bars represent rats that experienced TBI and were subsequently treated with vehicle control treatment. Right bars represent the rats that were treated with Alpha-1062 after experiencing TBI. The detailed treatment regimen and experimental setup can also be found in Example 1. The sham group comprised 8 rats, the vehicle and Alpha-1062 treatment groups comprised each 9 rats.

**Figure 7:** The graph shows the results of the Morris water maze test. The tests were performed as described in Example 1, wherein rats received either no treatment, vehicle treatment or Alpha-1062 treatment after an TBI event. The graph shows that animals that received Alpha-1062 after the TBI event performed from day 33 (D33) on significantly better than the vehicle treated animals. From day 34 on The Alpha-1062 treated rats found the submerged platform as well as the healthy (sham) animals. The left bars represent the healthy sham rats that experienced no TBI. Middle bars represent rats that experienced TBI and were subsequently treated with vehicle control treatment. Right bars represent the rats that were treated with Alpha-1062 after experiencing TBI. The detailed treatment regimen and experimental setup can also be found in Example 1. The sham group comprised 8 rats, the vehicle and Alpha-1062 treatment groups comprised each 9 rats.

**Figure 8:** The graph shows the results of the Morris water maze test, wherein the time in the correct quadrant (green line), the quadrant of the pool comprising the platform, was measured. The tests were performed as described in Example 1, wherein rats received either no treatment, vehicle treatment or Alpha-1062 treatment after an TBI event. The graph shows that animals that received Alpha-1062 after the TBI event performed from day 33 (D33) on significantly better than the vehicle treated animals. From days 33 on the Alpha-1062 treated animals spend an equivalent amount of time in the correct quadrant as the healthy (sham) animals. Both groups performed statistical significantly better than the vehicle treated animals who failed to show any preference for the correct quadrant. The left bars represent the healthy sham rats that experienced no TBI. Middle bars represent rats that experienced TBI and were subsequently treated with vehicle control treatment. Right bars represent the rats that were treated with Alpha-1062 after experiencing TBI. The detailed treatment regimen and experimental setup can also be found in Example 1. The sham group comprised 8 rats, the vehicle and Alpha-1062 treatment groups comprised each 9 rats.

**Figure 9:** The graph (top) shows the results of the new object recognition test. A schematic overview of the test is depicted on the bottom of the figure. Briefly, after habituation to the test room animals were confronted with two identical objects (A; left bars, and A'; center-left bars) where they became "familiar" with these objects. The same animals were later exposed to two objects (A = same as before; right bars, and B* = a novel/different object; yellow bars). Rats are naturally inquisitive and will, when healthy, spend more time exploring the new object (B*). Cognitively impaired animals fail to demonstrate this preference. Both Alpha-1062 treated animals (bars on the right side of the graph) and the healthy animals (sham; bars on the left of graph) demonstrated a statistically significant preference for the novel object. In contrast, the injured animals that received only vehicle treatment (bars in the middle of the graph), failed to demonstrate any preference for the novel object. The detailed treatment regimen and experimental setup can also be found in Example 1. The sham group comprised 8 rats, the vehicle and Alpha-1062 treatment groups comprised each 9 rats.

**Figure 10:** The graph shows the weight of the test animals throughout the study (day D0-D35). There was no significant difference in body weight among the three treatment groups at each time point during the study ($p>0.05$). The group of untreated animals (sham) shows from D20-35 vs D0 a significant body weight gain (D20-35 vs D0 within the group $p<0.05$). The group of vehicle treated animals shows from D1-6 compared to D0 a significant body weight loss and from D28-35 compared to D0 a significant body weight gain (within the group $p<0.05$). The group of Alpha-1062 treated animals shows from D1-7 compared to D0 a significant body weight loss and from D29-35 compared to D0 a significant body weight gain (within the group $p<0.05$). The detailed treatment regimen and experimental setup can also be found in Example 1. The sham group comprised 8 rats, the vehicle and Alpha-1062 treatment groups comprised each 9 rats.

## EXAMPLES

The invention is further described by the following examples. The examples are intended to further describe the invention by way of practical example and do not represent a limiting description of the invention.

**Example 1: Evaluating Efficacy of Alpha-1062 in Rats Following Traumatic Brain Injury**

In the present example the efficacy and use of the substances and pharmaceutical compositions according to one embodiment of the invention was performed in an exemplary 35-day study in a controlled cortical impact injury model. The aim of this study was to determine the efficacy of Alpha-1062 (galantamine benzoate gluconate salt) on functional (primary) and histological (secondary) outcome in young adult male rats after moderate traumatic brain injury (TBI) induced by controlled cortical impact (CCI).

The TBI model that was used in the present study were young adult (2-3 months) male Wistar rats subjected to moderate traumatic brain injury (TBI) induced by controlled cortical impact (CCI). Injury consisted of a single impact at 4.0 m/sec, 2.5 mm deformation to the left parietal cortex. In addition, a group of animals was left healthy / not injured to serve as a healthy control.

The animals were divided into 3 groups:

1) TBI + Vehicle (purified water, n=10)

2) TBI + Alpha-1062 (4.5 mg/kg) (n=10)

3) Healthy/not injured and not treated (Sham) (n=10)

To have 8 rats per study group, 10 rats (8/0.8=10) were included considering a 20% mortality. The total number of male rats used as planned were 30 male rats.

The substance used in this study was Alpha-1062 at 4.5 mg/kg. As control treatment (vehicle) purified water was administered intranasally (IN). The Alpha-1062 and the control treatment were initiated 2 hours after injury and continued twice daily (in an 6 hour interval) for 35 consecutive days.

In addition to the Alpha-1062 or control treatment, 1 day post injury Bromodeoxyuridine (BrdU) is administrated at 100 mg/kg per Intraperitoneal injection (IP) for 7 consecutive days to enable the monitoring of cell proliferation. Bromodeoxyuridine (BrdU) is a thymidine analog that incorporates DNA of dividing cells during the S-phase of the cell cycle. These experiments are ongoing.

Data were evaluated for normality applying the following statistical analyses: Data transformation was considered if the data was abnormal. The data was analyzed with GraphPad Prism Software 9.0. All data herein is presented as the mean $\pm$ standard deviation (SD) and were analyzed with one-way analysis of variance (ANOVA) or two-way ANOVA (treatment $\times$ time) followed by post hoc Tukey's multiple comparison test (more than two groups). Differences between means are considered statistically significant when $p$ is $< 0.05$.

During the in vivo part of the study cognitive functional tests were performed by conducting Morris water maze (MWM) tests and novel object recognition tests on all animals at 5 weeks after TBI, with the MWM test being performed 31-35 days after TBI (see Table 1).

In addition, neurological functional tests were performed by conducting footfault tests and observations to determine the modified neurological severity score (mNSS) at 1, 7, 14, 21, 28, 35 days after TBI (see Table 1). The mNSS is a composite score assessing motor, sensory, balance, and reflex functions. Details of the tests and scoring system can be found in Table 2.

Table 1: Tests performed at the primary endpoint of the study. mNSS: modified neurological severity score; MWM: Morris water maze; Experiments were performed in a blinded study regarding the treatment groups.

| Test/Day post TBI (mode rate) | 1d | 7d | 14d | 21d | 28d | 31-35d |
|---|---|---|---|---|---|---|
| Motor (Behavior: mNSS, footfault) | **x** | **x** | **x** | **x** | **x** | **x** |
| Spatial Cognition (MWM) | | | | | | **x** |
| Novel Object Recognition (NOR) | | | | | | **x** |

The Morris water maze (MWM) is a test used in behavioral neuroscience to study spatial learning and memory, for example in rats. It enables learning, memory, and spatial working to be studied, and can also be used to assess damage to particular cortical regions of the brain. The MWM can be used to assess the effect of lesions to the brain in areas concerned with memory, cognitive function and spatial learning. In the Morris water maze test a rat, for example,

is placed in a circular pool and is required to find an invisible, submerged platform that allows it to rest and leave the water. Usually, the rat is tracked while swimming in the pool and parameters, such as the time spend swimming in the quadrant of the pool comprising the platform or the time spend swimming in proximity to the platform and the total time spend until finding the platform (escape latency) are measured.

The adhesive removal test describes an experiment an adhesive tape is applied to each forepaw of an animal, e.g., a rat, and measuring the time-to-contact the adhesive, as well as the time-to-remove them. This test can be used to assess correct paw and mouth sensitivity (time-to-contact) and correct dexterity (time-to-remove).

The rat novel/new object recognition (NOR) assay is a relatively high-throughput, robust, and sensitive procedure for evaluating compounds for cognition-enhancing activity. For the test, rats are given the opportunity to explore two identical objects for a predetermined period of time. After a delay, the animals are then presented with two objects to explore, one of which is the same as in the first exploration trial, the other a new object. Depending on brain functionality, the rats will either explore the novel object for a greater time period, indicating memory for the familiar object, or will explore the novel and familiar objects for the same amount of time, indicating a lack of recall or loss of memory for the familiar object presented during the initial trial.

The modified neurological severity score (mNSS) is a rating scale for neurological functionality and includes a composite of motor (muscle status and abnormal movement), sensory (visual, tactile and proprioceptive), reflex and balance tests. The test is used to assess neurologic deficits and the grade of neurologic damage on the aspects of motor, ground walking, sensory, coordination of movements, reflex, and abnormal movements. It is usually performed with rodents, e.g. rats and the testers are blind to the treatment groups. Four aspects are observed, including abnormal movements or absence of reflex, beam balance test, sensory function, and locomotor function. The baseline for the normal animals is 0 points.

*A score value of 1 was given for the inability to perform a test, or for the lack of a tested reflex, or for abnormal movement.

Table 2: Modified Neurological Severity Score (mNSS): Tests & Scoring Values. *A score value of 1 was given for the inability to perform a test, or for the lack of a tested reflex, or for abnormal movement.

| **Motor test score values and descriptions (Normal score = 0; maximum possible summary score = 6)** | |
|---|---|
| 0 or 1* | Flexion of forelimb after raising rat by the tail |
| 0 or 1* | Flexion of hindlimb after raising rat by the tail |
| 0 or 1* | Head moved >10° to vertical axis within 30 seconds after raising rat by the tail |
| 0 | Normal walk after placing rat on the floor |
| 1 | Inability to walk straight after placing rat on the floor |
| 2 | Circling toward paretic side after placing rat on the floor |
| 3 | Falls down to paretic side after placing rat on the floor |
| **Sensory test score values and descriptions (Normal score = 0; maximum possible summary score = 2)** | |
| 0 or 1* | Placing test (visual and tactile test) |
| 0 or 1* | Procioceptive test (deep sensation,pushingpaw against table to stimulate limb muscles) |
| **Beam and balance test score values and descriptions (Normal score = 0; maximum possible summary score = 6)** | |
| 0 | Balances with steady posture |
| 1 | Grasps side of beam |
| 2 | Hugs beam and 1 limb falls down from beam |
| 3 | Hugs beam and 2 limbs fall down from beam, or spins on beam (60 seconds) |
| 4 | Attempts to balance on beam, but falls off (> 40 seconds) |
| *Score value 5 or for abnormal | of 1 was given for the inability to perform a test, or for the lack of a tested reflex, Attempts to balance on beam, but falls off (> 20 seconds) movement. |

(continued)

| Beam and balance test score values and descriptions (Normal score = 0; maximum possible summary score = 6) | |
|---|---|
| 6 | Falls off; no attempt to balance or hang on to beam (< 20 seconds) |
| **Reflex absence and, abnormal movements test score values and descriptions (Normal score = 0; maximum possible summary score = 4)** | |
| 0 or 1* | Pinna reflex (head shakes when auditory meatus is touched with cotton) |
| 0 or 1* | Corneal reflex (eye blink when cornea is lightly touched with cotton) |
| 0 or 1* | Startle reflex (motor response to a brief noise from snapping a clipboard paper) |
| 0 or 1* | Seizure, myoclonus, myodystony |

**Results**

Alpha -1062 administration acutely preserves and persistently improves recovery of locomotor skills in fore- and hindlimb foot-fault test

Animals were monitored as they walked across a mesh for the number of times their right forelimb "missed" proper placement, indicating reduced locomotor skill. This is indicative of trauma in the left side of the brain. The results are depicted in Figure 3. The tests were also carried out for the right hindlimb (Figure 4).

Alpha-1062 treatment (Figure 3 and Figure 4, gray bars) resulted in a statistically significant preservation of locomotor skill from the first day of treatment-and persistently statistically significantly improved recovery throughout the study, compared to the vehicle treated animals (Figure 3 and Figure 4, middle bars). By day 28 (D28) the performance of the Alpha-1062 treated animals was statistically indistinguishable from that of the uninjured (sham) animals (Figure 3 and Figure 4, right vs. left bars). The acute preservation of locomotor skill suggests that the early administration of Alpha-1062 post TBI, likely reduced the extent of the brain injury. Vehicle treated animals (Figure 3 and Figure 4, middle bars) show the natural levels of functional recovery after TBI.

Alpha-1062 administration improves outcomes in the modified neurological severity score (mNSS)

The mNSS is a composite score assessing motor, sensory, balance, and reflex functions (details of the tests and scoring system are found in Table 2). From day 14 (D14) of recovery onwards, Alpha-1062 treated animals performed statistically significantly better (Figure 5, right bars) than vehicle treated animals (Figure 5, middle bars).

Alpha-1062 administration improves motor skill in the right forelimb adhesive removal test

The results of the adhesive removal tests are shown in Figure 6. Starting on day 7 (D7) of recovery, and throughout the remainder of the study, Alpha-1062 treated animals (Figure 6, right bars) performed this motor skill as well as the uninjured (sham) animals (Figure 6, left bars).

Alpha-1062 administration improves spatial learning and memory in MWM test

The impact of Alpha-1062 treatment after TBI was assessed using the Morris water maze (MWM). The Morris water maze is a test of spatial learning for rodents that relies on distal cues to navigate from start locations around the perimeter of an open swimming arena to locate a submerged escape platform. Spatial learning is assessed across repeated trials and reference memory is determined by preference for the platform area ("correct quadrant") when the platform is absent. Figure 7 depicts the result of the final read out of the MWM tests in which the rats swam in a pool of water and the latency (time from start to goal = platform) to find the platform was assessed. On days 33 to 35 of (D33-D35) testing the Alpha-1062 treated animals (Figure 7, right bars) were able to find a hidden escape platform as well as the uninjured (sham) animals (Figure 7, left bars). Both groups performed statistically significantly better than the vehicle treated animals (middle bars).

In addition, the percentage of the time (% time) the rats spend in the "correct" quadrant of the pool, namely the quadrant where the platform was submerged in previous sessions, was also assessed. The results can be found in

Figure 8. On days 33 to 35 of testing the Alpha-1062 treated animals (Figure 8, right bars) spent an equivalent amount of time in the correct quadrant (enclosed by the green lines) as the uninjured (sham) animals (Figure 8, left bars). Both groups performed statistically significantly better than the vehicle treated animals (Figure 8, middle bars), who failed to show any preference for the correct quadrant throughout the test.

Alpha-1062 administration enables cognitive functional recovery post TBI

Object recognition tests were performed to assess the recovery of cognitive function after TBI. The test workflow is depicted at the bottom of Figure 9, the results, namely the percentage of time spend on exploring each object, are depicted at the top of Figure 9. Animals were placed in a room with two identical objects (A (left bars in top graph of Figure 9) & A' (middle bars) where they became "familiar" with these objects. The same animals were later exposed to two objects (A = same as before (right bars) and B = a novel/different object (yellow bars)). Rats are naturally inquisitive and will, when healthy, spend more time exploring the new object (B), which would be represented in the graph of Figure 9 by a high value for B (rights bars shown at top graph of Figure 9). Cognitively impaired animals fail to demonstrate this preference, as they do not remember the known object. Hence, a cognitive impairment could be assumed if a rat spends the same time exploring known and new objects. Both Alpha-1062 treated animals (Figure 9, bars on right side of top graph) and the uninjured animals (Figure 9, bars on left side) demonstrated a statistically significant preference for the novel object (B). In contrast, the injured animals that received only vehicle treatment (Figure 9, bars in the middle), failed to demonstrate any preference for the novel object.

Alpha-1062 administration does not alter body weight - no overt toxicity

As can be derived from Figure 10, rats treated with Alpha-1062 (Figure 10, right bars) experienced no significant alterations in body weight compared to vehicle treated or untreated animals (Figure 10, middle and left bars; no significant difference ($P>0.05$) in body weight among the 3 groups were detected at each time point). Only when compared to day zero of their own treatment group vehicle and Alpha-1062 treated animals showed a significant weight loss in the first week of treatment. Vehicle treated animals showed in comparison to day zero (D0) on days 1-6 (D1-6) significant body weight loss ($p<0.05$) but from days 28-35 (D28-35) a significant gain of body weight in comparison to day zero ($p<0.05$). Alpha-1062 treated animals showed in comparison to day zero (D0) on days 1-7 (D1-7) a significant body weight loss ($p<0.05$) but from days 29-35 (D29-35) a significant body weight gain in comparison to day zero. Untreated (sham) animals showed in comparison to their weight on day zero (D0) a significant ($P<0.05$) body weight gain between days 20-35 (D20-35) compared to day zero (D0).

Accordingly, Alpha-1062 administration did not show overt toxicity in treated animals when compared to vehicle animals.

As compared to the vehicle control, Alpha-1062 treatment significantly improved motor and sensory functional recovery in treated rats after TBI.

The experiments that assessed motor and sensory skills by foot fault, adhesive removal and mNSS tests showed an improved recovery of motor and sensory skills after TBI in Alpha-1062 treated rats. Also, NOR and MWM tests at 5 weeks after TBI showed significantly improved cognitive functional recovery in rats when Alpha-1062 treatment was administered. Alpha-1062 treatment did not significantly alter the body weight over the 35-day study, in comparison to untreated or vehicle treated animals. Accordingly, no evidence of toxicity in Alpha-1062 treated rats could be observed.

**Example 2: Antimicrobial properties of Alpha-1062**

In order to assess the anti-microbial properties of Alpha-1062, the USP (US Pharmacopeia) Chapter 51 Preservative Challenge Test (USP 51) was employed. The USP 51 is a common method used to gauge preservative effectiveness. Much like a Preservative Challenge Screen, it is used to evaluate the effect of preservatives in cosmetics, personal care products, and drug products. Preservatives are typically antimicrobial ingredients that are added to aqueous product formulations to help maintain the safety of the product by inhibiting the growth and reducing the number of microbial contaminants.

In the context of the present invention, Alpha-1062 (in the form of the gluconate salt) was assessed in place of (as) a preservative. No additional preservative was added to the Alpha-1062 gluconate preparation, rather an anti-microbial effect of the agent itself (Alpha-1062) was assessed. Alpha-1062 is therefore the anti-microbial agent in the assay and experiments described below.

The USP 51 challenge test utilizes 5 microorganisms (3 bacteria and 2 fungi) for challenge testing. Each of the microorganisms are known strains of pathogenic microorganisms and they represent a wide range of microbial physiologies.

Cultures of the following microorganisms were applied, as per standard guidelines for USP 51: Candida albicans

(ATCC No. 10231), Aspergillus brasiliensis (ATCC No. 16404), Escherichia coli (ATCC No. 8739), Pseudomonas aeruginosa (ATCC No. 9027), and Staphylococcus aureus (ATCC No. 6538).

The test was conducted in five sterile, capped bacteriological containers of suitable size into which a sufficient volume of product has been transferred. Each container was inoculated with one of the prepared and standardized inoculums and was mixed accordingly. The volume of the suspension inoculum used was between 0.5% and 1.0% of the volume of the product. The concentration of test microorganisms that was added to the product was such that the final concentration of the test preparation after inoculation was between $1 \times 10^5$ and $1 \times 10^6$ CFU per mL of the product, according to standard guidelines for Category 2 products.

The initial concentration of viable microorganisms in each test preparation was estimated based on the concentration of microorganisms in each of the standardized inoculums, as determined by the plate-count method.

The inoculated containers were incubated at 22.5 ± 2.5 °C and each container was sampled at the appropriate intervals, as specified in Table 3. Any changes observed in the appearance of the culture was recorded at these intervals. By using the plate-count procedure, the number of CFU present in each test preparation was determined for the applicable intervals.

An inactivator (neutralizer) of the specific antimicrobial was incorporated in the plate count or in the appropriate dilution prepared for plating. These conditions were determined in the validation study for that sample based upon the conditions of media and microbial recovery incubation times listed in Table 2. Using the calculated concentrations of CFU per mL present at the start of the test, the change in log10 values of the concentration of CFU per mL for each microorganism was calculated at the applicable test intervals, and the changes were expressed in terms of log reductions.

Antimicrobial Effectiveness Report for Alpha-1062 Gluconate:

| **Organism** | **Initial** | | **Day 14** | | **Day 28** | |
|---|---|---|---|---|---|---|
| | **CFU/mL** | **$Log_{10}$** | **CFU/mL** | **Log Reduction** | **CFU/mL** | **Log Reduction** |
| *E. coli* ATCC 8739 | $9.1\times10^5$ | 6.0 | <10 | 5.0 | <10 | 5.0 |
| *P. aeruginosa* ATCC 9027 | $2.9\times10^5$ | 5.5 | <10 | 4.5 | <10 | 4.5 |
| *S. aureus* ATCC 6538 | $4.0\times10^5$ | 5.6 | <10 | 4.6 | <10 | 4.6 |
| *C. albicans* ATCC 10231 | $6.7\times10^5$ | 5.8 | <10 | 4.8 | <10 | 4.8 |
| *A. brasiliensis* ATCC 16404 | $2.2\times10^5$ | 5.3 | $1.6 \times 10^2$ | 3.1 | $1.5 \times 10^2$ | 3.1 |

As can be seen from the data provided, the gluconate salt of Alpha-1062 exhibits a strong anti-microbial effect against all 5 organisms tested in the USP 51. The USP Criteria for Category 2 were employed in order to determine the presence of an anti-microbial effect. For bacteria, an antimicrobial effect is evident when at least a 2.0 log reduction from the initial count is evident at 14 days and no increase is evident from the 14-day count at 28 days. For yeast and molds, an antimicrobial effect is evident when no increase from the initial calculated count is determined at 14 and 28 days. With respect to the data above, the effect against bacteria and fungi therefore exceeded the requirements to demonstrate an anti-microbial effect for a Category 2 product.

## Claims

1. A pharmaceutical composition comprising a compound Alpha-1062 or salt thereof for use in the treatment of confirmed or suspected traumatic brain injury (TBI) in a subject.

2. The pharmaceutical composition for use according to claim 1, wherein the composition is in liquid form, preferably a solution, emulsion or a suspension.

3. The pharmaceutical composition for use according to claim 2, wherein the composition is administered intranasally.

4. The pharmaceutical composition for use according to any one of the preceding claims, wherein the composition is

self-preserving and anti-microbial, wherein the composition is absent of additional antimicrobial preservatives.

5. The pharmaceutical composition for use according to any one of the preceding claims, wherein the composition is present in a multi-use dispenser configured for intranasal administration.

6. The pharmaceutical composition for use according to any one of the preceding claims, wherein the subject that has or is suspected to suffer from TBI shows one or more symptoms selected from the group consisting of dizziness, balance problems, headaches, nausea, vomiting, light sensitivity, impaired memory, sleep abnormalities, impaired concentration and impaired vision.

7. The pharmaceutical composition for use according to any one of the preceding claims, wherein the subject that has or is suspected to suffer from moderate or severe TBI shows one or more symptoms selected from the group consisting of weakness in arms and legs, balance and coordination problems, severe or increasing headaches, impaired sensory perception, impaired cognitive skills, impaired memory, impaired communication and learning, personality changes, behavior abnormalities and impaired vision and hearing.

8. The pharmaceutical composition for use according to any one of the preceding claims, wherein the subject that has or is suspected to suffer from severe TBI shows one or more symptoms selected from the group consisting of paralysis, coma, loss of consciousness, dilated pupils, loss of cerebrospinal fluid from the ears or nose, loss of bowel and/or bladder control, breathing problems, slow pulse, breathing problems, slow breathing rate with an increase in blood pressure and droopy eyelid or facial weakness.

9. The pharmaceutical composition for use according to any one of the preceding claims, wherein the traumatic brain injury (TBI) leads to or is accompanied by one or more injuries selected from the group consisting of a hematoma, contusion, intracerebral hemorrhage, subarachnoid hemorrhage, diffuse injuries, diffuse axonal injury, ischemia, primary brain injury and secondary brain injury.

10. The pharmaceutical composition for use according to any one of the preceding claims, wherein the traumatic brain injury (TBI) is caused by a closed head injury or penetrating head injury.

11. The pharmaceutical composition for use according to any one of the preceding claims, wherein the traumatic brain injury (TBI) is caused by a head injury resulting from an incident selected from the group consisting of a fall, a motor vehicle-related incident, a strike or blow to the head from or against an object, a sports-related incident, interpersonal physical violence or violence by other means.

12. The pharmaceutical composition for use according to any one of the preceding claims, wherein the subject that has or is suspected to suffer from TBI is an infant under the age of 4 years, preferably under 12 months of age, or is a child from 4 to 12 years of age, or an adolescent from 12 to 17 years of age.

13. The pharmaceutical composition for use according to any one of claims 1 to 11, wherein the subject that has or is suspected to suffer from TBI is an adult, selected from an adult of between 18 and 65 years of age, or an elderly adult of over 65 years of age.

14. The pharmaceutical composition for use according to any one of the preceding claims, wherein the traumatic brain injury (TBI) is associated with trauma to the nasal cavity, and/or for use in the treatment of traumatic brain injury (TBI) and concurrent treatment and/or prevention of microbial infection of the nasal cavity.

15. The pharmaceutical composition for use according to any one of the preceding claims, wherein the traumatic brain injury (TBI) is associated with disruption of the blood brain barrier, and/or for use in the treatment of traumatic brain injury (TBI) and concurrent treatment and/or prevention of microbial infection of the central nervous system.

16. The pharmaceutical composition for use according to any one of the preceding claims, wherein the compound Alpha-1062 or salt thereof is present at a concentration of 1 to 200 mg/mL, preferably at a concentration of 5 to 100 mg/mL.

17. The pharmaceutical composition for use according to any one of the preceding claims, wherein the compound Alpha-1062 is present from a gluconate salt at a concentration of 50 to 100 mg/mL.

18. The pharmaceutical composition for use according to any one of the preceding claims, wherein the composition is

administered at a dose of 0.1 to 200 mg, preferably 1 to 100 mg, more preferably 2 to 40 mg, one to three times daily.

**19.** The pharmaceutical composition for use according to any one of the preceding claims, wherein the composition is administered within 24 hours of injury, preferably within 1 hour of injury.

Fig. 1

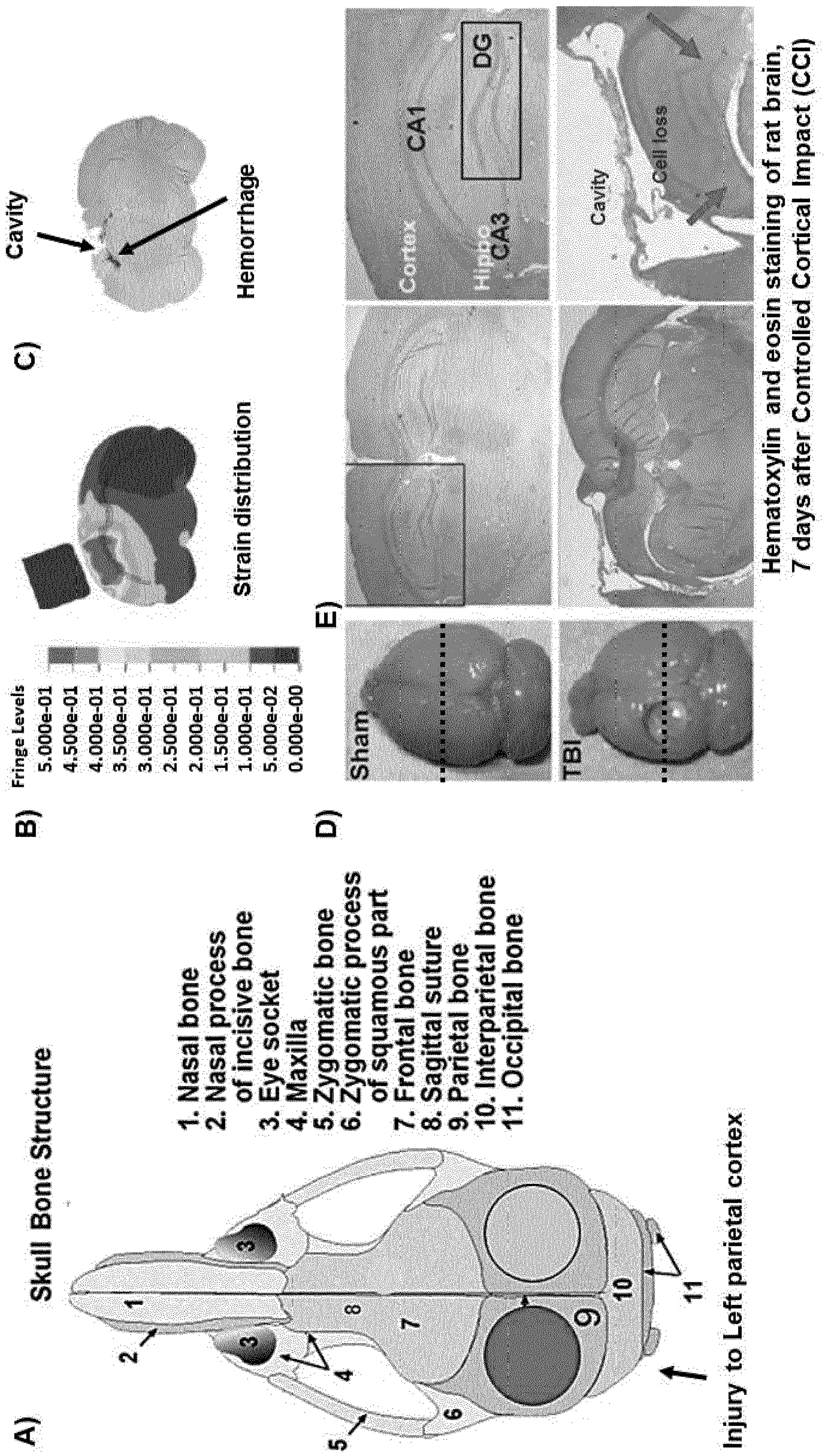
A)
Skull Bone Structure
1. Nasal bone
2. Nasal process of incisive bone
3. Eye socket
4. Maxilla
5. Zygomatic bone
6. Zygomatic process of squamous part
7. Frontal bone
8. Sagittal suture
9. Parietal bone
10. Interparietal bone
11. Occipital bone
Injury to Left parietal cortex
B)
Fringe Levels
5.000e-01
4.500e-01
4.000e-01
3.500e-01
3.000e-01
2.500e-01
2.000e-01
1.500e-01
1.000e-01
5.000e-02
0.000e-00
Strain distribution
C)
Cavity
Hemorrhage
D)
Sham
TBI
E)
Cortex
Hippo
CA1
CA3
DG
Cavity
Cell loss
Hematoxylin and eosin staining of rat brain,
7 days after Controlled Cortical Impact (CCI)

Fig. 2

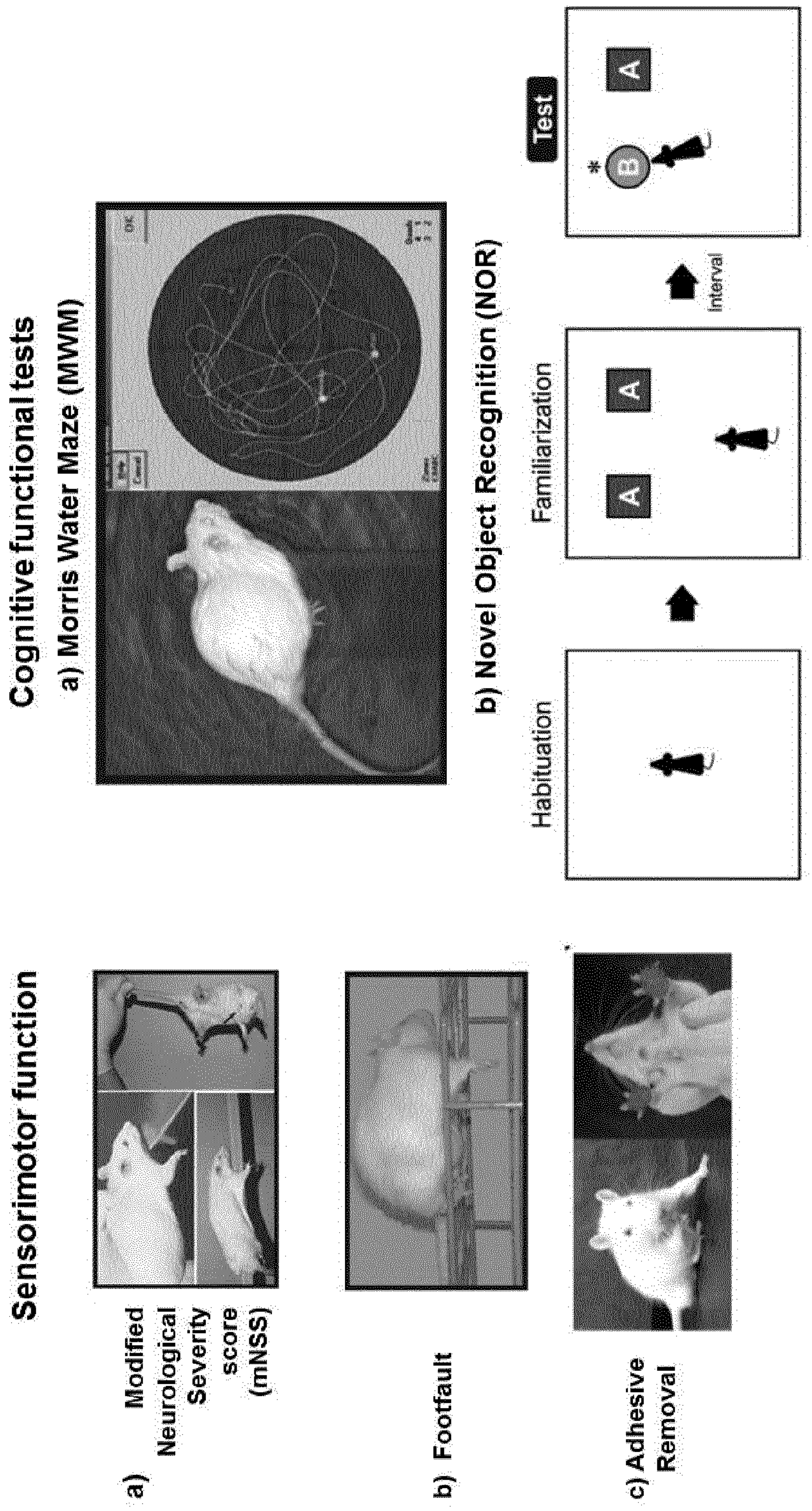
Sensorimotor function
a) Modified Neurological Severity score (mNSS)
b) Footfault
c) Adhesive Removal
Cognitive functional tests
a) Morris Water Maze (MWM)
b) Novel Object Recognition (NOR)
Habituation
Familiarization
Interval
Test
A
B
*

Fig. 3

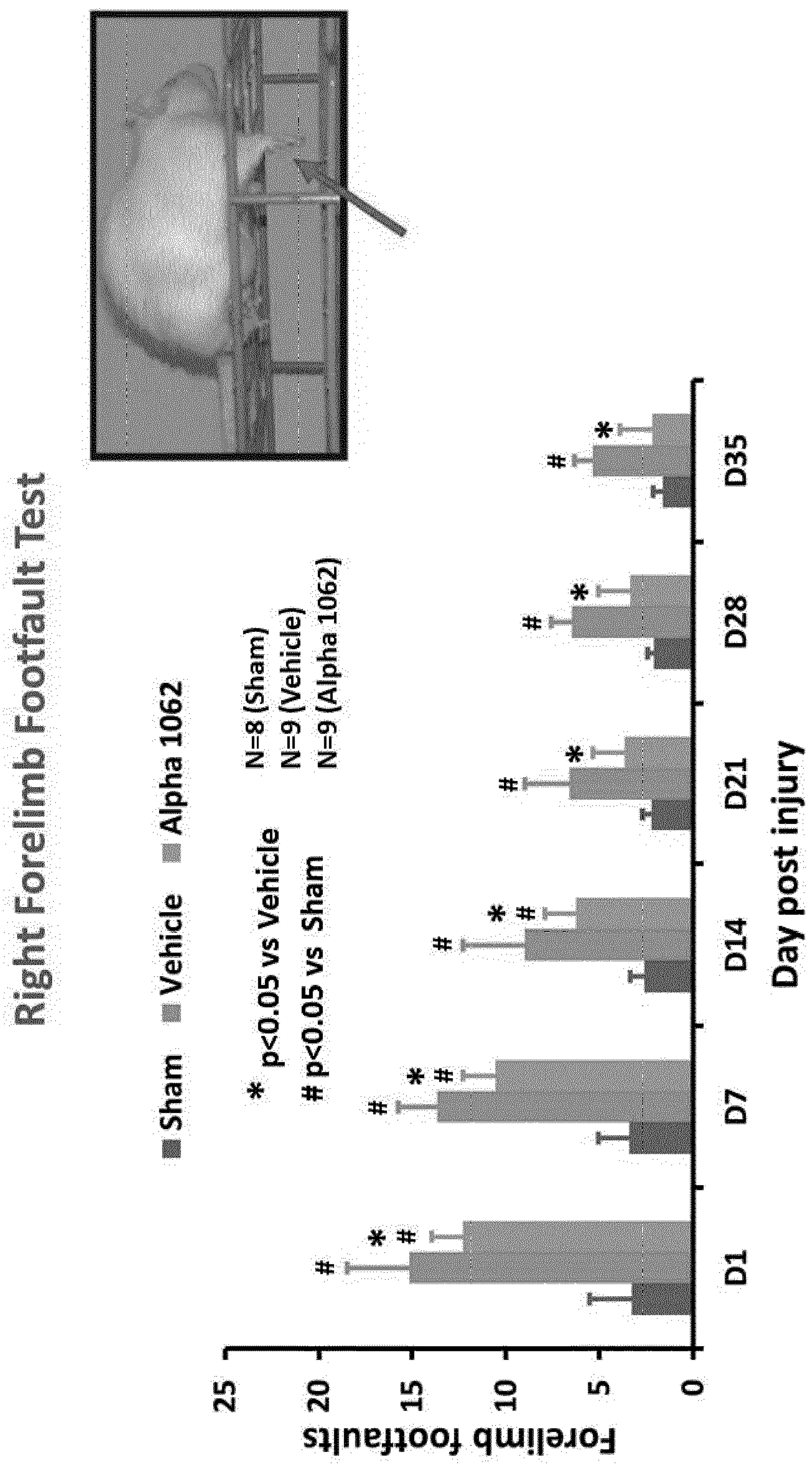
Right Forelimb Footfault Test
Sham
Vehicle
Alpha 1062
* p<0.05 vs Vehicle
# p<0.05 vs Sham
N=8 (Sham)
N=9 (Vehicle)
N=9 (Alpha 1062)
Forelimb footfaults
25
20
15
10
5
0
D1
D7
D14
D21
D28
D35
Day post injury

Fig. 4

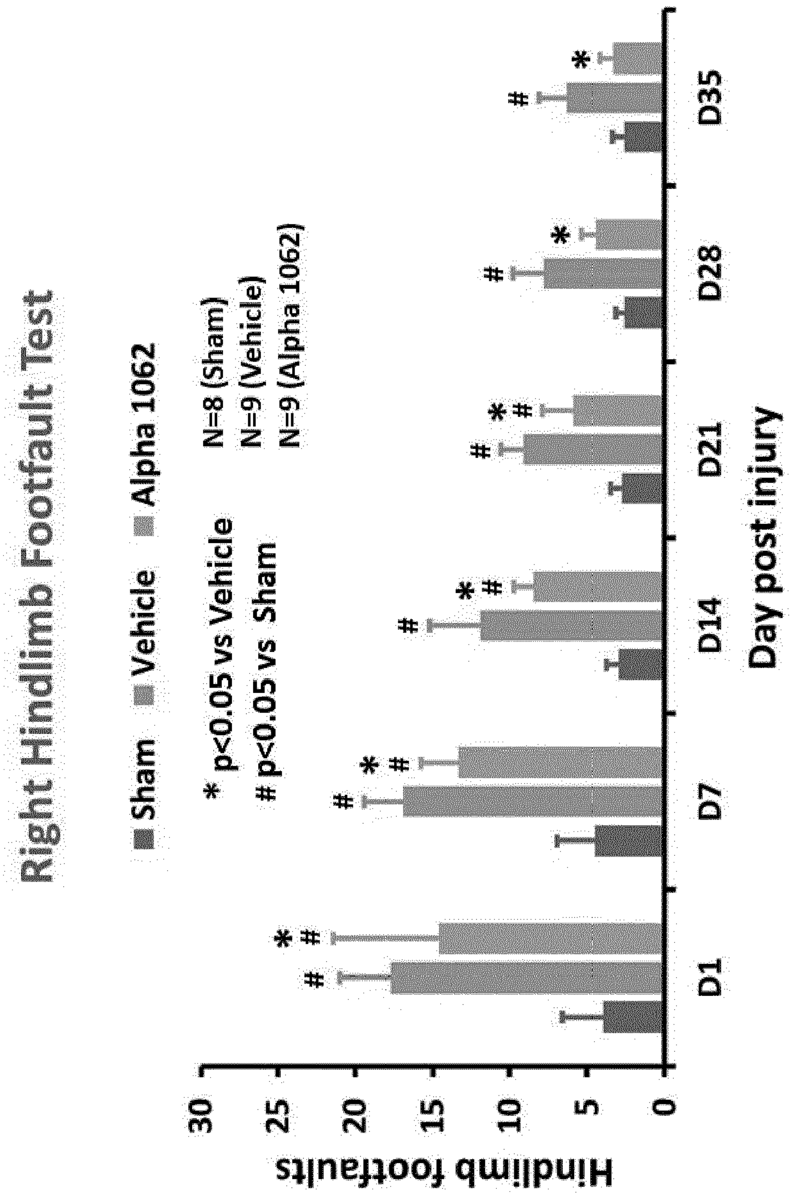
Right Hindlimb Footfault Test
Sham
Vehicle
Alpha 1062
* p<0.05 vs Vehicle
# p<0.05 vs Sham
N=8 (Sham)
N=9 (Vehicle)
N=9 (Alpha 1062)
Hindlimb footfaults
0
5
10
15
20
25
30
D1
D7
D14
D21
D28
D35
Day post injury

Fig. 5

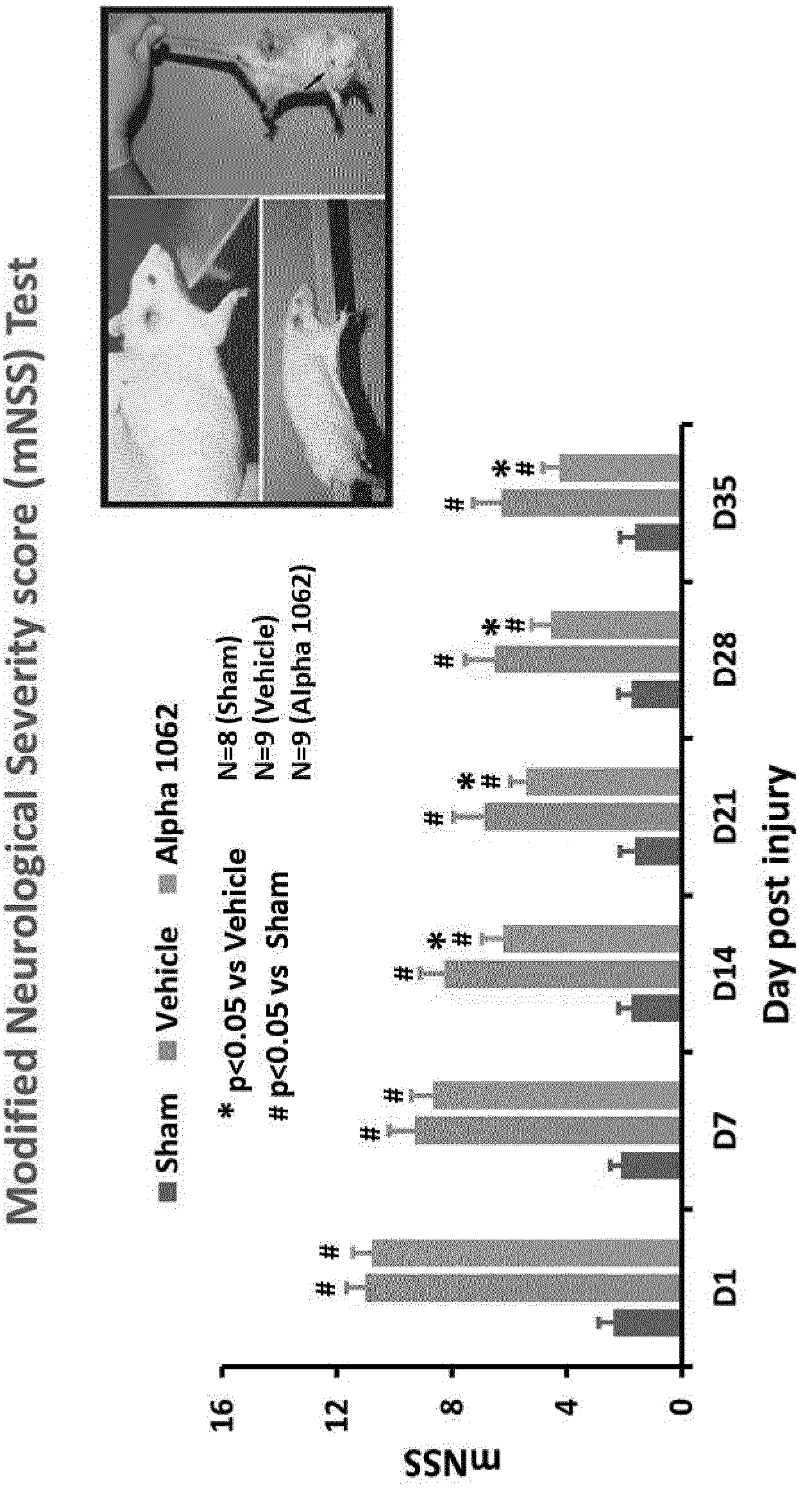
Modified Neurological Severity score (mNSS) Test
Sham
Vehicle
Alpha 1062
* p<0.05 vs Vehicle
# p<0.05 vs Sham
N=8 (Sham)
N=9 (Vehicle)
N=9 (Alpha 1062)
mNSS
0
4
8
12
16
D1
D7
D14
D21
D28
D35
Day post injury

Fig. 6

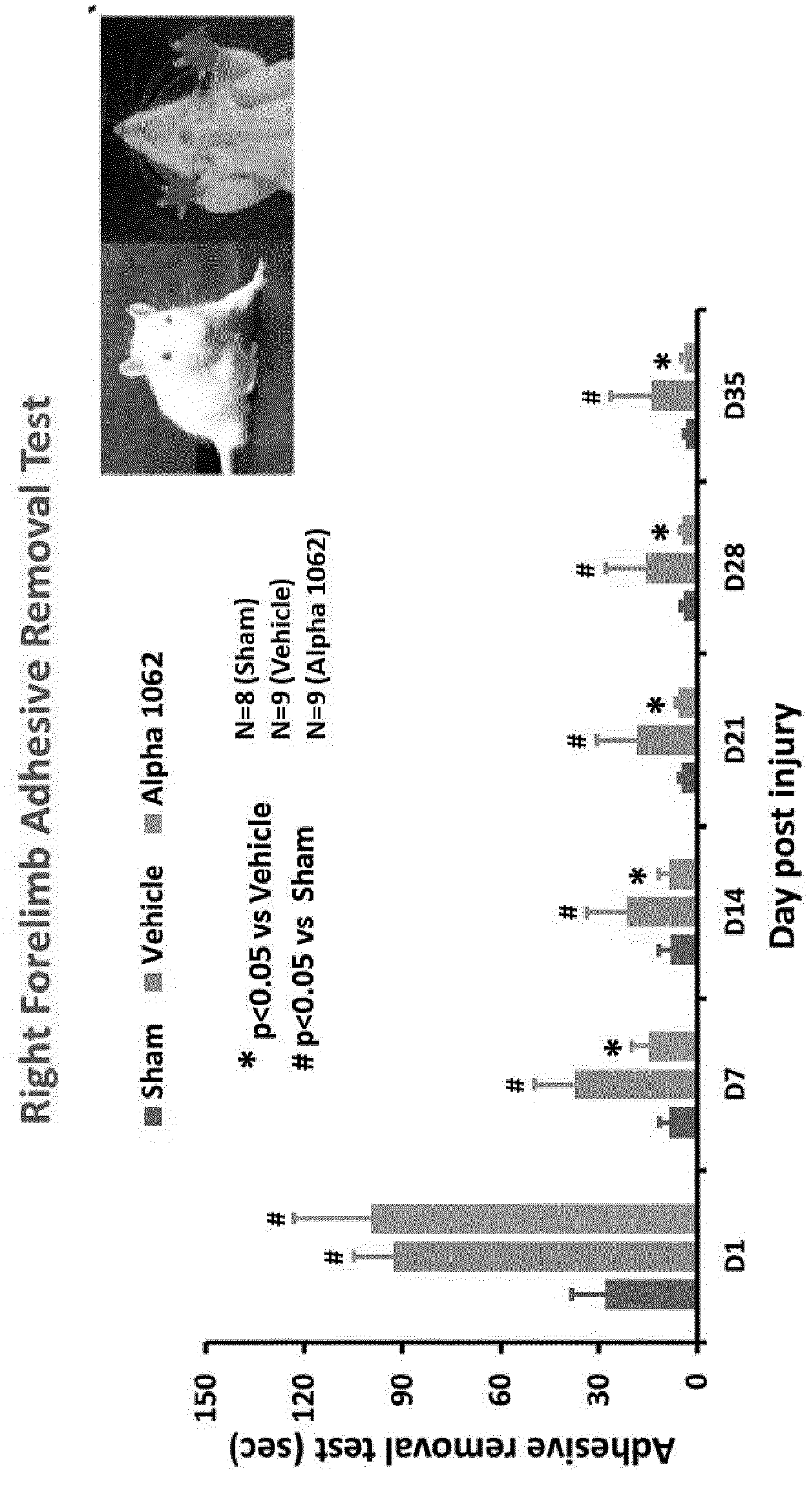
Right Forelimb Adhesive Removal Test
Sham
Vehicle
Alpha 1062
* p<0.05 vs Vehicle
# p<0.05 vs Sham
N=8 (Sham)
N=9 (Vehicle)
N=9 (Alpha 1062)
Adhesive removal test (sec)
150
120
90
60
30
0
D1
D7
D14
D21
D28
D35
Day post injury

Fig. 7

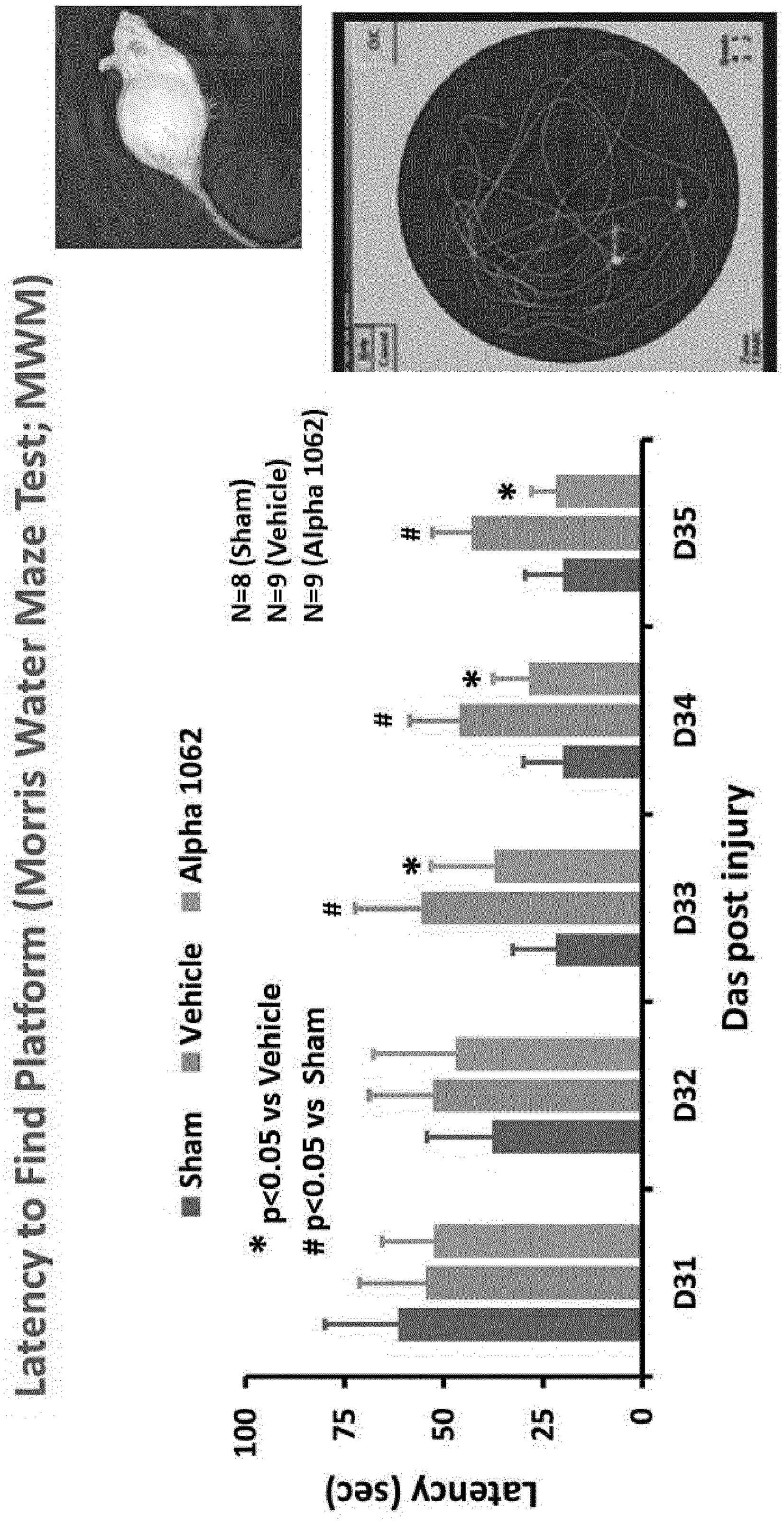
Latency to Find Platform (Morris Water Maze Test; MWM)
Sham
Vehicle
Alpha 1062
* p<0.05 vs Vehicle
# p<0.05 vs Sham
N=8 (Sham)
N=9 (Vehicle)
N=9 (Alpha 1062)
Latency (sec)
100
75
50
25
0
D31
D32
D33
D34
D35
Das post injury

Fig. 8

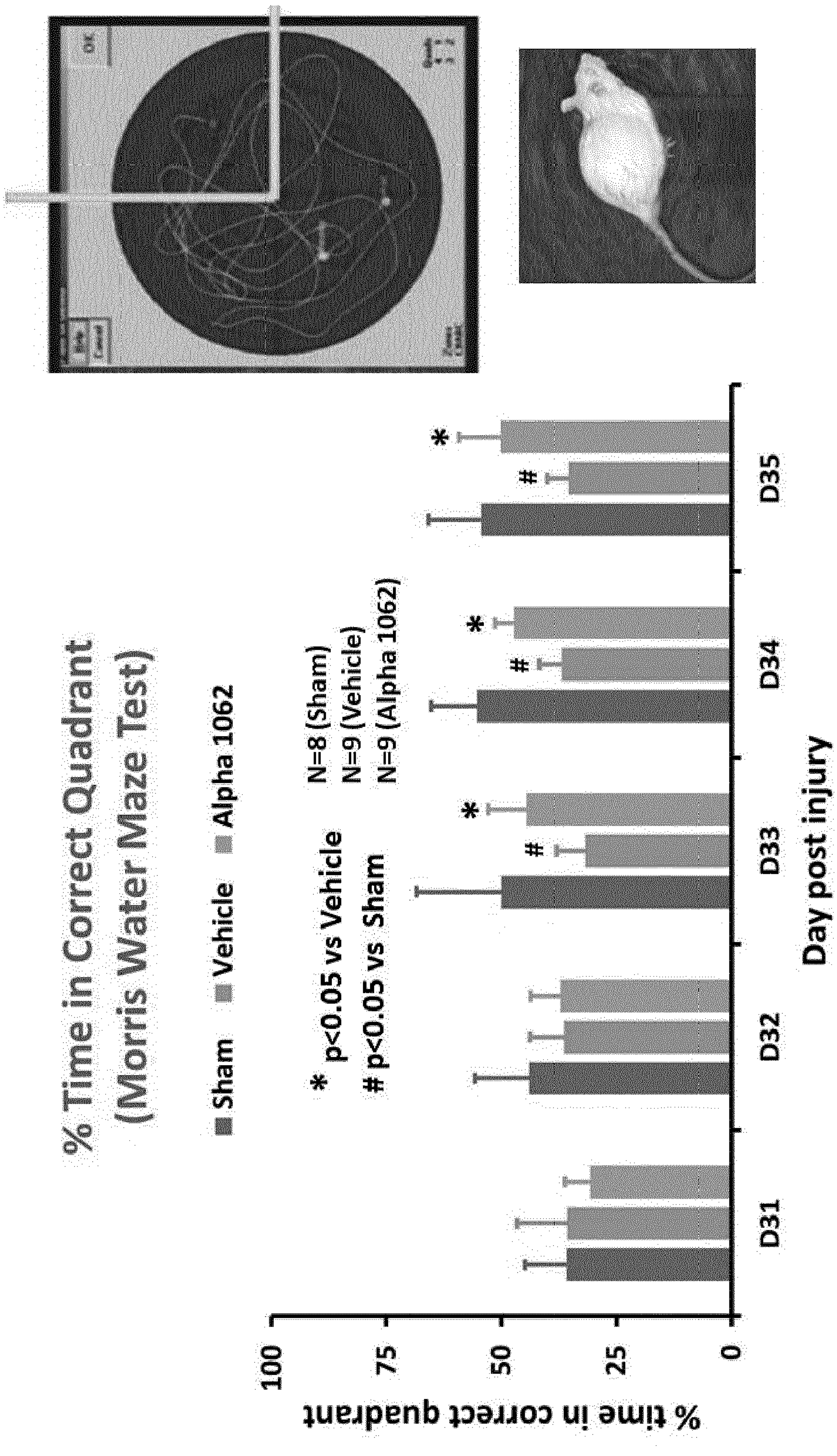
% Time in Correct Quadrant
(Morris Water Maze Test)
Sham
Vehicle
Alpha 1062
* p<0.05 vs Vehicle
# p<0.05 vs Sham
N=8 (Sham)
N=9 (Vehicle)
N=9 (Alpha 1062)
% time in correct quadrant
100
75
50
25
0
D31
D32
D33
D34
D35
Day post injury

Fig. 9

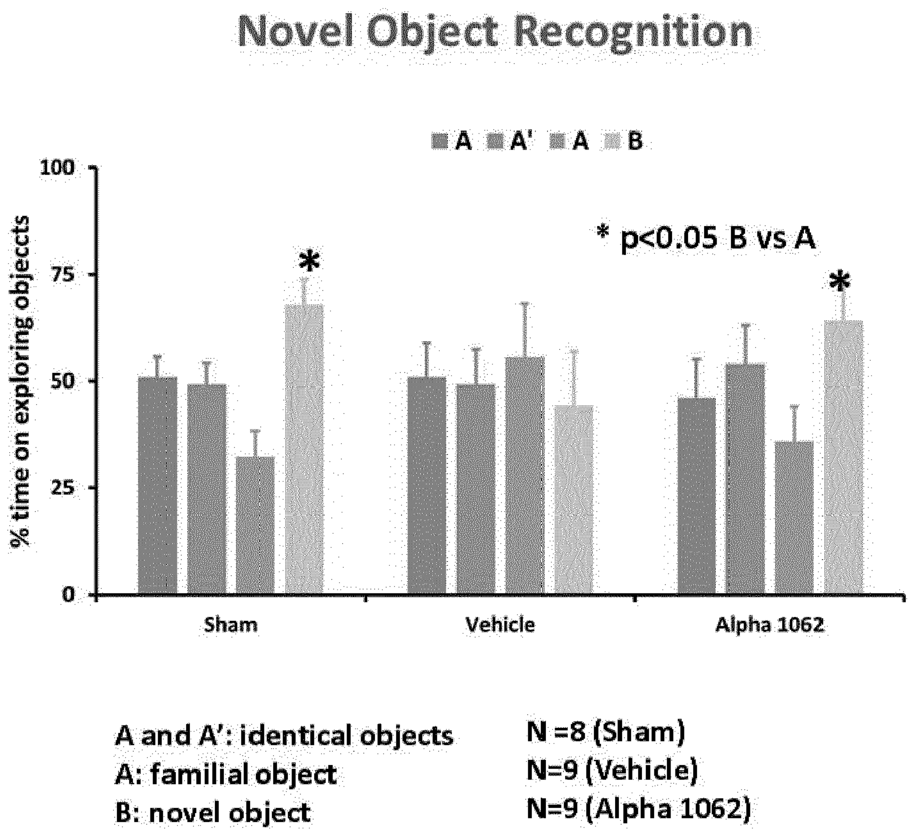
Novel Object Recognition
A
A'
A
B
100
75
50
25
0
% time on exploring objeccts
* p<0.05 B vs A
*
*
Sham
Vehicle
Alpha 1062
A and A': identical objects
A: familial object
B: novel object
N =8 (Sham)
N=9 (Vehicle)
N=9 (Alpha 1062)

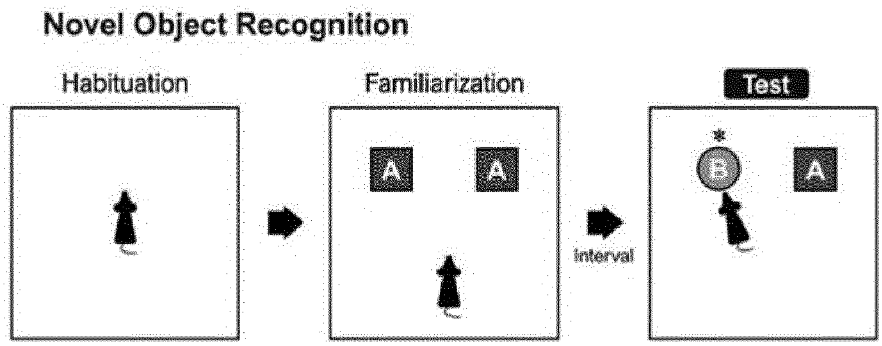
Novel Object Recognition
Habituation
Familiarization
Test
A
A
Interval
*
B
A

Fig. 10

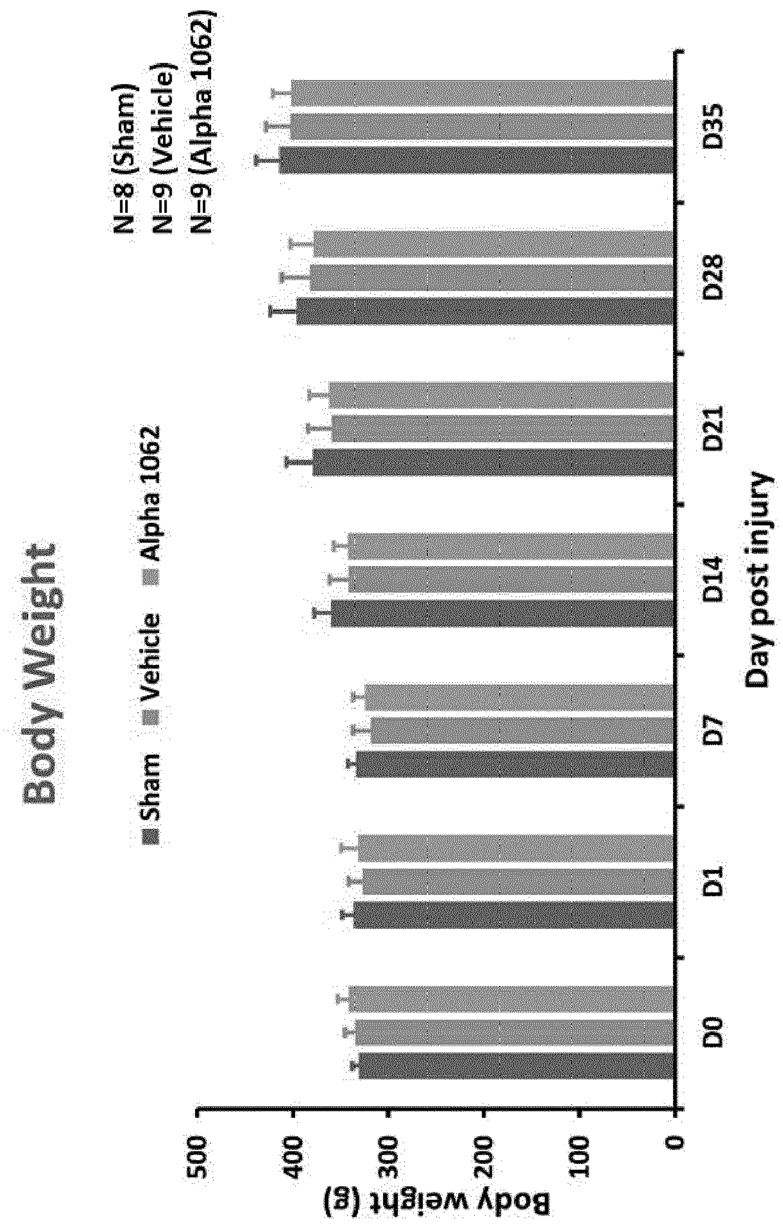
Body Weight
Sham
Vehicle
Alpha 1062
N=8 (Sham)
N=9 (Vehicle)
N=9 (Alpha 1062)
Body weight (g)
500
400
300
200
100
0
D0
D1
D7
D14
D21
D28
D35
Day post injury

Europäisches Patentamt
European Patent Office
Office européen des brevets

# EUROPEAN SEARCH REPORT

Application Number
EP 21 21 0661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TENOVUO ET AL: "Central acetylcholinesterase inhibitors in the treatment of chronic traumatic brain injury-clinical experience in 111 patients", PROGRESS IN NEURO-PSYCHOPHARMACOLOGY & BIOLOGICAL PSYCHIATRY, ELSEVIER, GB, vol. 29, no. 1, 1 January 2005 (2005-01-01), pages 61-67, XP027798437, ISSN: 0278-5846 [retrieved on 2005-01-01] * abstract * * page 62, right-hand column, paragraph 2.2; tables 1-3 * * page 65, left-hand column, paragraphs 3.4.2, 3.4.3 - page 65, right-hand column * ----- | 1-19 | INV. A61K31/55 A61P25/28 A61P43/00 |
| Y | ALFRED MAELICKE ET AL: "Memogain is a Galantamine Pro-drug having Dramatically Reduced Adverse Effects and Enhanced Efficacy", JOURNAL OF MOLECULAR NEUROSCIENCE, vol. 40, no. 1-2, 1 January 2010 (2010-01-01), pages 135-137, XP055041228, ISSN: 0895-8696, DOI: 10.1007/s12031-009-9269-5 * abstract * ----- | 1-19 | TECHNICAL FIELDS SEARCHED (IPC): A61K A61P |

-/--

The present search report has been drawn up for all claims

| Place of search | Date of completion of the search | Examiner |
|---|---|---|
| Munich | 4 May 2022 | Madalinska, K |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 21 0661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BAAKMAN ANNE C. ET AL: "First in human study with a prodrug of galantamine: Improved benefit-risk ratio?", ALZHEIMER'S & DEMENTIA: TRANSLATIONAL RESEARCH & CLINICAL INTERVENTIONS, vol. 2, no. 1, 1 January 2016 (2016-01-01), pages 13-22, XP055917563, ISSN: 2352-8737, DOI: 10.1016/j.trci.2015.12.003 Retrieved from the Internet: URL:https://www.ncbi.nlm.nih.gov/pmc/articles/PMC5644278/pdf/main.pdf> * abstract * | 1-19 | |
| Y | US 11 077 119 B2 (NEURODYN LIFE SCIENCES INC [CA]) 3 August 2021 (2021-08-03) * abstract; figures 7,9,13-14 * * the whole document * * column 11, line 59 - column 12, line 3 * | 1-19 | |
| Y | DATABASE Biomedical Literature Lis [Online] 1 January 2010 (2010-01-01), "Memogain: A novel drug candidate with improved efficacy and reduced adverse effects", XP007914738, retrieved from Biomedical Literature List * abstract * | 1-19 | TECHNICAL FIELDS SEARCHED (IPC) |

-/--

The present search report has been drawn up for all claims

| Place of search | Date of completion of the search | Examiner |
|---|---|---|
| Munich | 4 May 2022 | Madalinska, K |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 21 21 0661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KALETTA T ET AL: "Memogain, a novel high potency drug treatment for AD", ABSTRACTS OF THE ANNUAL MEETING OF THE SOCIETY FOR NEUROSCIENCE, SOCIETY FOR NEUROSCIENCE, US, vol. 40, 1 January 2010 (2010-01-01), XP009163806, ISSN: 0190-5295<br>* abstract *<br>----- | 1-19 | |

TECHNICAL FIELDS SEARCHED (IPC)

The present search report has been drawn up for all claims

1

| Place of search | Date of completion of the search | Examiner |
|---|---|---|
| Munich | 4 May 2022 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO.

EP 21 21 0661

This annex lists the patent family members relating to the patent documents cited in the above-mentioned European search report. The members are as contained in the European Patent Office EDP file on The European Patent Office is in no way liable for these particulars which are merely given for the purpose of information.

04-05-2022

| Patent document cited in search report | Publication date | Patent family member(s) | Publication date |
|---|---|---|---|
| US 11077119 B2 | 03-08-2021 | ES 2880399 T3 | 24-11-2021 |
| | | HK 1257737 A1 | 25-10-2019 |
| | | JP 6799648 B2 | 16-12-2020 |
| | | JP 2019214595 A | 19-12-2019 |
| | | PT 3417862 T | 06-08-2021 |
| | | US 2019262352 A1 | 29-08-2019 |
| | | US 2021322437 A1 | 21-10-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1940817 A
- WO 2009127218 A
- US 20090253654 A
- WO 2014016430 A
- US 9238532 B
- US 9345616 B
- US 20150043958 A
- US 8827124 B
- US 8986266 B
- US 20140231536 A1
- US 9095864 B
- US 20090294347 A


### Non-patent literature cited in the description


- **HAARBAUER-KRUPA et al.** *J Neurotrauma,* 2021
- **BRAZINOVA et al.** *J Neurotrauma,* 2021
- **LOY C et al.** Galantamine for Alzheimer's disease and mild cognitive impairment. *Cochrane Database of Systematic Reviews,* 2006
- **MARX ; BIRKHOFF.** Multi-Dose Container for Nasal and Ophthalmic Drugs: A Preservative Free Future?. *Drug Development - A Case Study Based Insight into Modern Strategies,* 2011
- **SHOJAEI A.** Buccal mucosa as a route for systemic drug delivery: a review. *J Pharm Pharmaceut Sci,* 1998, vol. 1, 15-30